# EUROPEAN PATENT APPLICATION

(11) **EP 3 705 055 A1**
(43) Date of publication of application: **09.09.2020**
(21) Application number: 20161545.7
(22) Date of filing: 06.03.2020
(51) Int. Cl.: A61B 17/072, A61B 90/00, A61B 17/29, A61B 17/00

(54) **SURGICAL STAPLER WITH SMALL DIAMETER ENDOSCOPIC PORTION**

(30) Priority: 08.03.2019 US 201916297055
(71) Applicant: Covidien LP, Mansfield, MA 02048 (US)
(72) Inventor: WHITFIELD, Kenneth H., North Haven, Connecticut 06473 (US); CASASANTA, Jr., Thomas, Southington, Connecticut 06489 (US); EBNER, Timothy, Southington, Connecticut 06489 (US); GADDY, Anthony, Windsor, Connecticut 06095 (US)
(74) Representative: Maschio, Antonio

(57) **Abstract**

A surgical stapler including a shipping lock configured to obstruct movement of a drive member is provided. The surgical stapler includes an elongate body, a tool assembly pivotally secured to the elongate body, a drive member movable within the tool assembly between retracted and advanced positions, and a shipping lock releasably secured to the elongate body. The shipping lock includes a projection. When the shipping lock is secured to the elongate body, the projection obstructs movement of the drive member to its advanced position.

## Description

### CROSS-REFERENCE TO RELATED APPLICATIONS

The present application is a continuation-in-part of U.S. Patent Application Serial No. 15/440,010, filed on February 23, 2017, the content of which is incorporated by reference herein in its entirety.

### BACKGROUND

### Technical Field

The present disclosure relates to surgical staplers, and, more particularly, to endoscopic surgical staplers including small diameter endoscopic portions suitable for performing endoscopic surgical procedures including, *inter alia,* pediatric and thoracic surgical procedures.

### Background of Related Art

Surgical staplers that effect simultaneous dissection and suturing of tissue are well known in the art. The advent of surgical staplers has increased the speed of tissue suturing and thus, increased the speed of surgical procedures to reduce patient trauma.

Endoscopic surgical staplers for suturing tissue endoscopically through small incisions in the skin or through a cannula rather than by performing open surgical procedures are also well known in the art and have also reduced patient trauma.

Typically endoscopic surgical staplers include an elongated body that supports a tool assembly. The elongated body and tool assembly (endoscopic portion) are dimensioned to pass through the small incision in the skin or the cannula. It is advantageous to minimize the dimensions of the elongated body and the tool assembly to minimize trauma to the patient. Thus, a continuing need exists for small diameter surgical staplers suitable for endoscopic use.

### SUMMARY

In accordance with the present disclosure, a surgical stapler is provided that includes a body portion that has a distal portion of a reduced diameter to facilitate insertion of the endoscopic portion through a small diameter trocar assembly. The surgical stapler includes an actuation device, and a stapler reload releasably secured to the actuation device. The stapler reload includes a body portion, a tool assembly, and a drive assembly movable within tool assembly. The body portion includes a large diameter portion defining a first diameter and a small diameter portion defining a second diameter extending distally from the large diameter portion. The tool assembly is supported on a distal portion of the small diameter portion. The small diameter portion is dimensioned to pass through an 8mm trocar. The surgical stapler further includes a shipping lock releasably secured to the distal portion of the small diameter portion of the body portion. The shipping lock is engageable with the drive assembly for preventing longitudinal movement of the drive assembly.

In embodiments, the shipping lock is configured to prevent articulation of the tool assembly relative to the body portion. The shipping lock may include a locking portion that engages the tool assembly to prevent articulation of the tool assembly relative to the body portion. The tool assembly may include an anvil assembly and a cartridge assembly supporting a plurality of staples. The drive assembly may be movable through the tool assembly to eject the plurality of staples from the cartridge assembly.

The drive assembly may include a clamping member and the shipping lock may include a projection. The projection may engage the clamping member when the shipping lock is secured to the body portion to obstruct advancement of the drive assembly. The clamping member may include an upper flange. The projection of the shipping lock may engage the upper flange to prevent advancement of the drive assembly. The shipping lock may include curved arms configured to frictionally engage the body portion of the stapler reload.

Also provided is a surgical stapler with a shipping lock configured to obstruct movement of a drive member. The surgical stapler includes an elongate body, a tool assembly pivotally secured to the elongate body, a drive member movable within the tool assembly between retracted and advanced positions, and a shipping lock releasably secured to the elongate body. The shipping lock includes a projection. When the shipping lock is secured to the elongate body, the projection obstructs movement of the drive member to its advanced position.

In embodiments, the shipping lock is configured to prevent the tool assembly from pivoting relative to the body portion. The shipping lock may include a locking portion that engages the tool assembly to prevent pivoting of the tool assembly relative to the body portion. The tool assembly may include an anvil assembly and a cartridge assembly supporting a plurality of staples and the drive assembly is movable through the tool assembly to eject the plurality of staples from the cartridge assembly.

The drive assembly may include a clamping member. The projection may be positioned to obstruct movement of the clamping member towards the advanced position when the shipping lock is secured to the body portion. The clamping member may include an upper flange. The projection of the shipping lock may be positioned to engage the upper flange to obstruct movement of the drive member to the advanced position. The shipping lock may include curved arms configured to frictionally engage the elongate body.

### BRIEF DESCRIPTION OF THE DRAWINGS

Various embodiments of the presently disclosed surgical stapler with small diameter endoscopic portion are described herein with reference to the drawings, wherein:
FIG. 1 is a side, perspective view of an embodiment of the presently disclosed surgical stapler with a small diameter endoscopic portion including a stapler reload and an adapter assembly secured to a handle assembly, with a tool assembly of the stapler reload in an open, non-articulated position;
FIG. 2 is a side, perspective view of an adapter assembly secured to a powered actuation device for use with the stapler reload of FIG. 1;
FIG. 3 is a first side perspective view of the stapler reload shown in FIG. 2;
FIG. 4 is a second side perspective view of the stapler reload shown in FIG. 2;
FIG. 5 is an exploded perspective view of the stapler reload shown in FIG. 2;
FIG. 6 is a cross-sectional view taken along section line 6-6 of FIG. 3;
FIG. 7 is an enlarged view of the indicated area of detail shown in FIG. 6;
FIG. 8 is a cross-sectional view taken along section line 8-8 of FIG. 7;
FIG. 9 is a cross-sectional view taken along section line 9-9 of FIG. 8;
FIG. 10 is a perspective view of a large diameter portion of a body portion of the stapler reload of FIG. 1, with an upper half-section removed and a rotatable sleeve in a first or locked position;
FIG. 11 is a cross-sectional view taken along section line 11-11 of FIG. 7;
FIG. 12 is a side perspective view of the large diameter portion shown in FIG. 10 with an outer tube removed and the rotatable sleeve in the first position;
FIG. 13 is a side perspective view of the large diameter portion shown in FIG. 10 with the outer tube removed and the rotatable sleeve in a second or unlocked position;
FIG. 14 is a cross-sectional view taken along section line 14-14 of FIG. 7, with a locking member in a proximal position and a shipping lock secured to the body portion of the stapler reload;
FIG. 15 is a cross-sectional view taken along section line 15-15 shown in FIG. 7, with the locking member in a distal position as the shipping lock is being released from the body portion of the stapler reload;
FIG. 16 is a cross-sectional view taken along section line 16-16 shown in FIG. 7, with the shipping lock removed;
FIG. 17 is a cross-sectional view taken along section line 17-17 shown in FIG. 11;
FIG. 18 is a perspective view of the large diameter portion shown in FIG. 10, with the upper half-section removed and the rotatable sleeve in the second position;
FIG. 19 is a perspective top view of the large diameter portion shown in FIG. 18, with a lockout assembly in a first or unlocked condition;
FIG. 20 is a cross-sectional view of the large diameter portion shown in FIG. 19, subsequent to actuation of the stapler reload;
FIG. 21 is a perspective top view of the large diameter portion shown in FIG. 19, subsequent to actuation of the stapler reload;
FIG. 22 is a perspective view of the large diameter portion shown in FIG. 21, during a second actuation of the stapler reload;
FIG. 23 is a perspective top view of a proximal portion of the large diameter portion shown in FIGS. 18-21, with a lockout member and lockout member tool shown in phantom;
FIG. 24 is a cross-sectional top view of the stapler reload shown in FIGS. 1 and 2;
FIG. 25 is a cross-sectional top view of the large diameter portion shown in FIGS. 18-21, prior to actuation of the stapler reload;
FIG. 26 is a cross-sectional top view of a small diameter portion of the stapler reload shown in FIGS. 1 and 2;
FIG. 27 is a perspective view of a proximal portion of an inner body of the small diameter portion shown in FIG. 26;
FIG. 28 is a perspective view of the proximal portion of the inner body shown in FIG. 27, with the outer tube removed;
FIG. 29 is an enlarged perspective view of a pnuematic seal according to an embodiment of the present disclosure;
FIG. 30 is a perspective view of an articulating portion of the presently disclosed stapler reload including a mounting assembly;
FIG. 31 is a perspective view of a coupling member according to an embodiment of the present disclosure;
FIG. 32 is a perspective view of the coupling member shown in FIG. 31 and an upper mounting bracket of the mounting assembly shown in FIG. 30;
FIG. 33 is a perspective view of the coupling member and upper mounting bracket shown in FIG. 32, prior to locking the coupling member to the upper mounting bracket;
FIG. 34 is a perspective view of the coupling member and upper mounting bracket shown in FIGS. 32 and 33, subsequent to locking the coupling member to the upper mounting bracket;
FIG. 35 is an enlarged section view of the indicated area of detail shown in FIG. 5;
FIG. 36 is a perspective view of the articulating portion of the stapler reload shown in FIG. 30 with the upper mounting bracket of the mounting assembly and the cartridge assembly removed;
FIG. 37 is a perspective view of the tool assembly the stapler reload shown in FIGS. 1 and 2;
FIG. 38 is a perspective, top view of the distal portion of the small diameter portion of the stapler reload shown in FIGS. 1 and 2, with the upper mounting bracket and upper housing half-section removed;
FIG. 39 is a perspective, side view of the articulating portion of the stapler reload shown in FIG. 30, with the outer tube of the body portion and the channel of the cartridge assembly removed;
FIG. 40 is a perspective view of the tool assembly shown in FIG. 37, with the channel of the cartridge assembly removed;
FIG. 41 is a perspective view of the cartridge assembly shown in FIG. 40, with parts separated;
FIG. 42 is a perspective, top view of the body portion of the stapler reload shown in FIGS. 1 and 2, with the upper housing half-section and proximal tube body removed;
FIG. 43 is a perspective view of the articulating portion of the stapler reload shown in FIG. 30, with the outer tube of the small diameter portion removed;
FIG. 44 is a perspective view of the assembled articulating portion of the stapler reload shown in FIG. 43.
FIG. 45 is a side perspective view of a stapler reload according to another embodiment of the present disclosure including a shipping lock;
FIG. 46 is an enlarged side perspective view of a distal portion of the stapler reload and shipping lock shown in FIG. 45;
FIG. 47 is an exploded perspective view of a proximal portion of the stapler reload shown in FIG. 45;
FIG. 48 is a cross-sectional view taken along section line 48-48 of FIG. 45;
FIG. 49 is a cross-sectional view taken along section line 49-49 of FIG. 48; and
FIG. 50 is a side perspective view of the distal portion of the stapler reload and shipping lock shown in FIG. 46.

### DETAILED DESCRIPTION OF EMBODIMENTS

Embodiments of the presently disclosed surgical stapler with a small diameter endoscopic portion will now be described in detail with reference to the drawings wherein like reference numerals designate identical or corresponding elements in each of the several views. In this description, the term "proximal" is used generally to refer to the portion of the apparatus that is closer to a clinician, while the term "distal" is used generally to refer to the portion of the stapler that is farther from the clinician. In addition, the term "endoscopic" is used generally to refer to surgical procedures performed through a small incision or a cannula inserted into a patient's body including endoscopic, laparoscopic and arthroscopic surgical procedures. Finally, the term clinician is used generally to refer to medical personnel including doctors, nurses, and support personnel.

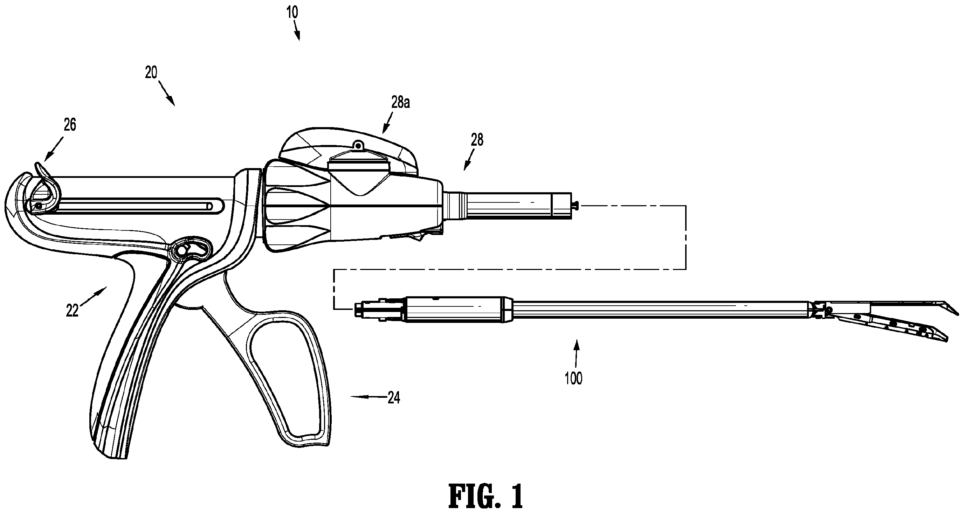

FIG. 1 illustrates an embodiment of the presently disclosed small diameter reload is shown generally as stapler reload 100. The stapler reload 100 is part of a surgical stapling device 10 including a handle assembly or actuation device 20. In embodiments, the stapler reload 100 may be integrally formed with the handle assembly 20. As shown, the handle assembly 20 includes a handle 22, a trigger member 24 pivotally secured to the handle 22, and a pair of firing levers 26 (only one shown) extending from the handle 22, and an elongated body 28 rotatably secured to the handle 22 and including an articulating lever 28a. For a detailed description of an exemplary handle assembly, please refer to commonly owned U.S. Patent No. 8,070,033, the content of which is incorporated herein by reference in its entirety.

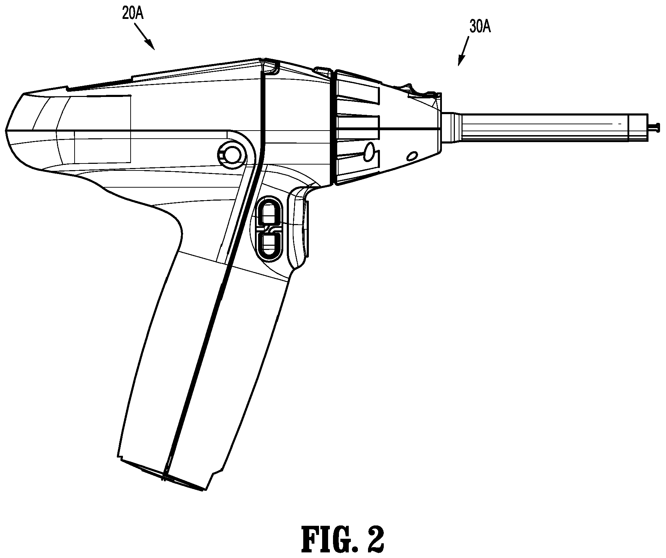

Turning briefly to FIG. 2, it is envisioned that the stapler reload 100 may be operated with a powered handle assembly 20A. As shown, an adapter assembly 30A is secured to the powered handle assembly 20A and the stapler reload 100 is secured to the adapter assembly 30A. It is envisioned that the adapter assembly 30A and the stapler reload 100 (FIG. 1) and/or the handle assembly 20A can be integrally formed such that the stapler reload 100 is non-releasably supported on, or forms an integral extension of, the adapter assembly 30A and/or the handle assembly 20A. For a detailed description of exemplary handle assemblies and adapter assemblies, please refer to commonly owned U.S. Patent Application Publication No. 2012/0253329 ("the '329 publication"), the content of which is incorporated by reference herein in its entirety.

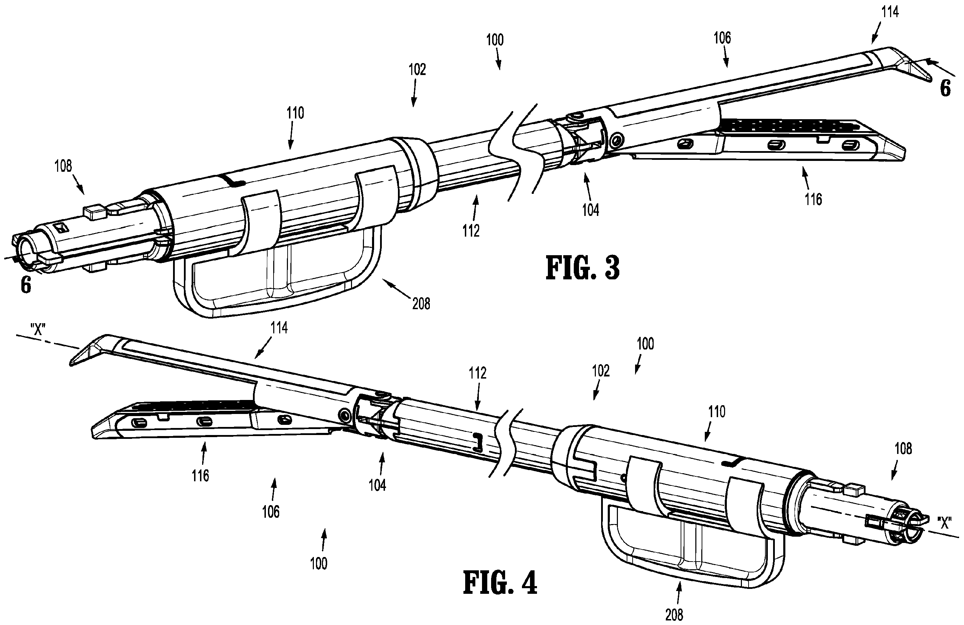

Referring to FIGS. 3 and 4, the stapler reload 100 includes a body portion 102, a mounting assembly 104, and a tool assembly 106. The body portion 102 defines a longitudinal axis "x" that is aligned with the longitudinal axis of the adapter assembly 30 (FIG. 1) and has a coupling portion 108, a large diameter portion 110, and an endoscopic or small diameter portion 112. The large diameter portion 110 of the stapler reload 100 has a diameter greater than the diameter of the small diameter portion 112. In embodiments, the small diameter portion 112 of the stapler reload 100 is dimensioned to be received in an 8mm trocar assembly (not shown), whereas the large diameter portion 110 is about 12mm in diameter and dimensioned to support a lock assembly 200 and a lockout assembly 220, as discussed in detail below.

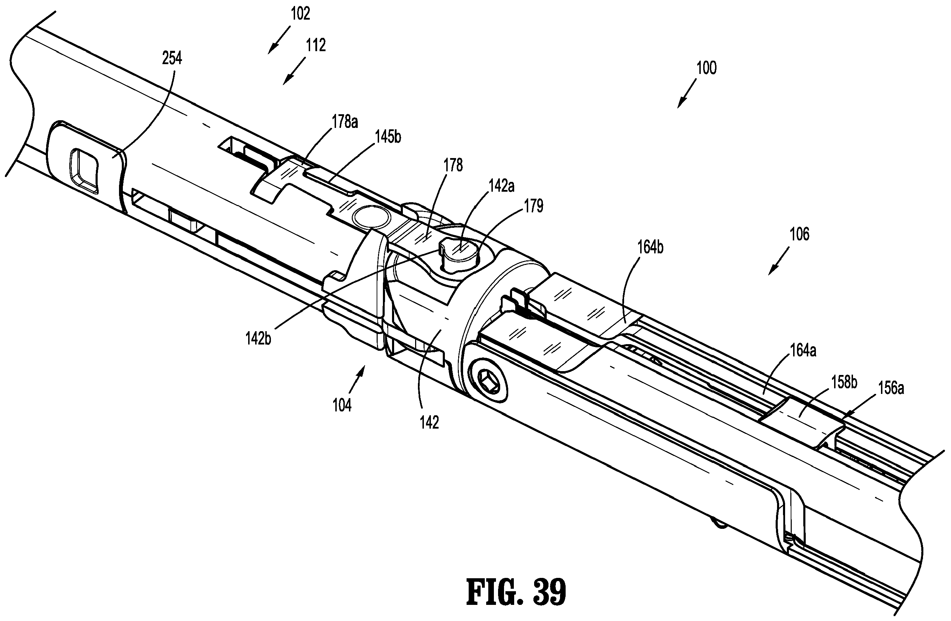

The tool assembly 106 includes an anvil assembly 114 and a cartridge assembly 116. In embodiments, the cartridge assembly 116 is pivotally supported in relation to the anvil assembly 114 and is movable between an open position (FIG. 2) and a closed or approximated position (FIG. 39). Alternately, the anvil 114 can be pivotally coupled to the cartridge assembly 116. The mounting assembly 104 is supported on a distal portion of the body portion 102 and pivotally supports the tool assembly 106 to facilitate articulation of the tool assembly 106 about an axis perpendicular to the longitudinal axis "x" of the body portion 102 between an articulated position in which a longitudinal axis of the tool assembly 106 defines an acute angle with the longitudinal axis "x" of the body portion 102 and a non-articulated position in which the longitudinal axes of the tool assembly 106 and the body portion 102 are aligned.

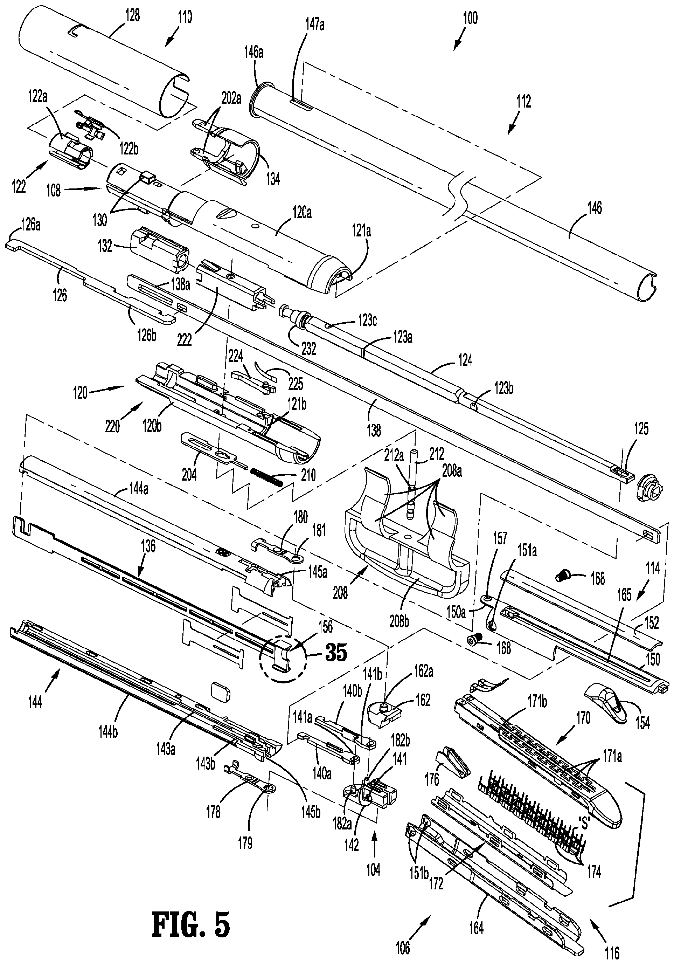

Referring to FIG. 5, the large diameter portion 110 of the body portion 102 of the stapler reload 100 includes an inner housing 120 having an upper housing half-section 120a and a lower housing half-section 120b that are secured relative to each other with a tab and slot configuration as shown, or in any other suitable manner.. The upper and lower housing half-sections 120a, 120b define channels 121a, 121b which slidably receive a drive member 124 and a first articulation link 126. The upper and lower housing half-sections 120a, 120b are received within a proximal body tube 128 that prevents separation of the half sections 120a, 120b.

The proximal portion of the upper housing half-section 120a defines the coupling portion 108 and includes engagement nubs 130 for releasably engaging the distal portion of the adapter assembly 30 (FIG. 1) in a bayonet-type coupling arrangement. For a detailed description of an exemplary coupling portion please refer to commonly owned U.S. Patent No. 5,865,361 ("the '361 patent"), the content of which is incorporated herein by reference in its entirety.

An electrical connector assembly 122 is supported within the proximal portion of the upper housing half-section 120a of the body portion 102 of the stapler reload 100. The connector assembly 122 includes a connector base 122a and a contact member 122b. For a detailed discussion of an exemplary electrical connection assembly, please refer to commonly own U.S. Pat. App. Pub. No. 2016/0249929 ("the '929 application"), the content of which is incorporated by reference herein in its entirety.

The drive member 124 of the stapler reload 100 includes a proximal portion that supports a drive block 132. The drive block 132 is configured to releasably engage a control rod 32 (FIG. 2) of the adapter assembly 30 (FIG. 2) to translate movement of the control rod 32 into movement of the drive member 124. The drive block 132 is operably engaged by a locking sleeve 134 to prevent firing of the stapler reload 100 prior to the stapler reload 100 being properly secured to adapter assembly 20 (FIG. 1). A distal portion of the drive member 124 defines a slot 125 that engages a hooked proximal portion of a drive assembly 136 such that distal movement of the drive member 124 effects distal movement of the drive assembly 136.

By providing the stapler reload 100 with a large diameter portion 110 for housing the drive block 132 and the locking sleeve 134, as well as other components of the stapler 10 described below, the diameter of the small diameter portion 112 can be minimized, for example, to about 8mm such that it may be received within an 8mm trocar assembly (not shown), while the large diameter portion 110 may be about 12 mm in diameter or larger.

Referring still to FIG. 5, the first articulation link 126 defines a hooked proximal portion 126a that is configured to engage an articulation shaft (not shown) of the adapter assembly 30 (FIG. 1) when the stapler reload 100 is secured to the adapter assembly 30. A distal portion 126b of the first articulation link 126 engages a proximal portion 138a of a second articulation link 138. Although shown having a hook and notch configuration and a corresponding slotted configuration, the respective distal portion 126b of the first articulation link 126 and the proximal portion 138a of the second articulation link 138 may be secured to one another in any suitable manner.

The proximal portion 138a of the second articulation link 138 is slidably positioned within the large diameter portion 110 of the body portion 102 of the stapler reload 100. The distal portion 138b of the second articulation link 138 engages a first articulation member 140a which is pivotally connected to a lower mounting bracket 142 of the mounting assembly 104 at a location offset from the longitudinal axis of the body portion 102 such that longitudinal movement of the second articulation link 138 effects pivotal movement of the tool assembly 106 about the longitudinal axis.

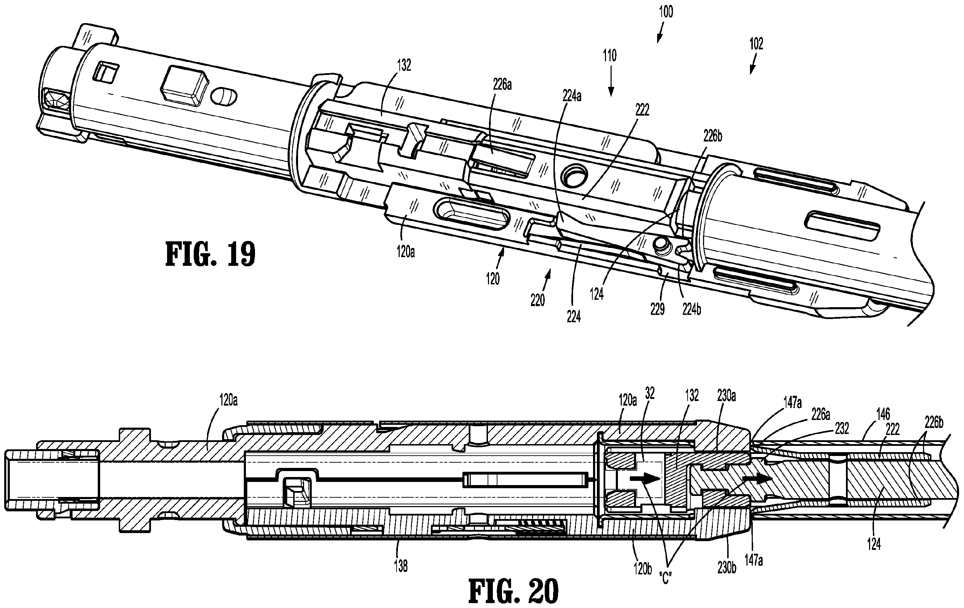

The small diameter portion 112 of the body portion 102 of the stapler reload 100 includes an inner body 144 having upper and lower half-sections 144a, 144b that are received within an outer tube 146. The upper and lower half-sections 144a, 144b define a first channel 143a that slideably receives the drive member 124 and the drive assembly 136. The upper and lower half-sections 144a, 144b also define a second channel 143b that slideably receives the second articulation link 138. Respective distal ends of the upper and lower half-sections 144a, 144b also define cutouts 145a, 145b. The outer tube 146 includes a proximal flange 146a for securing the outer tube 146 to the upper and lower housing half-section 120a, 120b of the large diameter portion 110 of the body portion 102. The outer tube 146 defines openings 147a, 147b (FIG. 20) for receiving respective protrusions 230a, 230b (FIG. 20) of respective upper and lower housing half-sections 120a, 120b.

As noted above, the tool assembly 106 includes the anvil assembly 114 and the cartridge assembly 116. The anvil assembly 114 includes an anvil body 150 and an anvil cover 152 which is secured to the topside of the anvil body 150 to define a channel (not shown). In embodiments, staple pockets 151 are formed directly into the anvil body 150. Alternatively, the anvil body 150 may be provided with an anvil plate (not shown). In embodiments, the anvil body 150 may be one-piece, e.g., monolithic. In embodiments, the staple pockets may be formed using micro-electrolytic dissolution (MED). In embodiments, the anvil body 150 may include a lubricated hard coating.

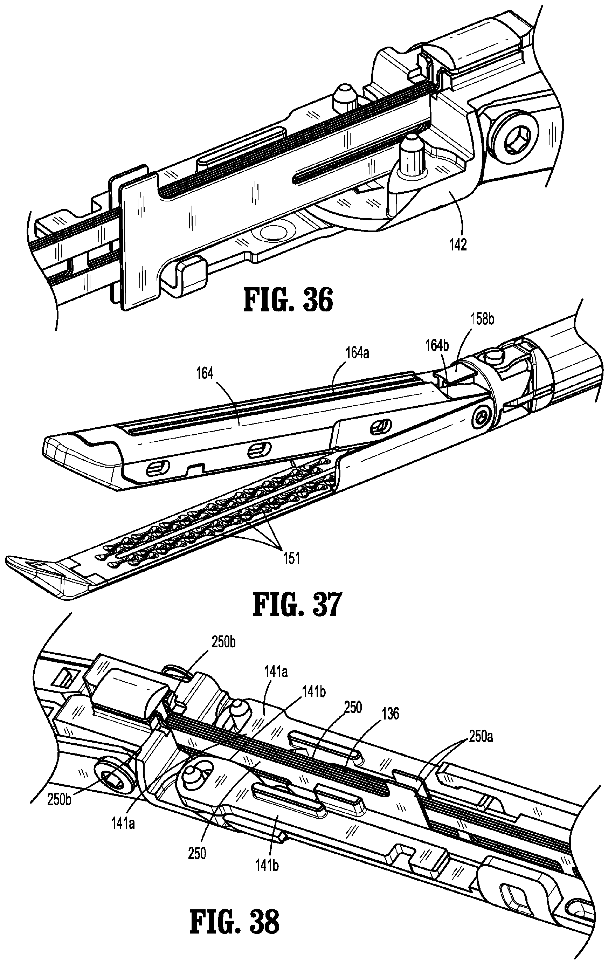

In embodiments, a dissector tip 154 is supported on the distal portion of the anvil body 150. The anvil body 150 defines plurality of staple receiving depressions 151 (FIG. 37) and a longitudinal slot 153 which is dimensioned to slideably receive a portion of a working portion 156 of the drive assembly 136 of the stapler reload 100. A proximal portion of the anvil body 150 includes a bracket 150a defining a hole 157 for receiving a pivot member or boss 162a of an upper mounting bracket 162 of the mounting assembly 104.

In embodiments, the tissue dissector 154 is secured to a distal portion of the anvil assembly 114 with friction fit, adhesives, welding, mechanical fasteners, or in any suitable manner. The tissue dissector 154 has a tapered configuration and extends distally of the distal portion of the cartridge assembly 116 to allow the tool assembly 106 to be manipulated about tissue adjacent a surgical site. The tissue dissector 154 may be attached to the distal portion of the anvil assembly 114, as shown, or may be integrally and/or monolithically formed with the anvil assembly 114. Alternately, the tissue dissector 154 may be attached to the cartridge assembly 116. In addition, the anvil assembly 114 may include a buttress material (not shown) to strengthen tissue being sutured and dissected as is known in the art.

In some embodiments, the cartridge assembly 116 includes a channel 164 and a staple cartridge 166 that is received within the channel 164. It is envisioned that the staple cartridge 166 may be attachable to the channel 164 by a snap-fit connection, or in any suitable manner, and may be removable to permit replacement following a stapling procedure. For a detailed description of an exemplary replaceable staple cartridge assembly, please refer commonly owned U.S. Pat. App. Pub. No. 2012/0286021 ("the '021 publication"), the content of which is incorporated herein by reference in its entirety.

The channel 164 is pivotally secured to the anvil body 150 by pivot pins 168 which extend through openings 151a formed in the anvil body 150 and openings 151b formed in the channel 164. The staple cartridge 166 includes a cartridge body 170 which may define only two rows of staple retention slots 171a on each side of a knife slot 171b to facilitate reduction in the diameter of the tool assembly 106. The knife slot 171b is aligned with an elongated slot 165 defined in the channel 164 to facilitate passage of a dynamic clamping member 156a. The staple retention slots 171a are positioned along a tissue contact surface of the cartridge body 170 and are aligned with the staple forming depressions 151 (FIG. 37) of the anvil body 150. Each staple retention slot 171a is configured to receive a fastener or staple "S" and a pusher 174. A cartridge shield 172 allows the pushers 174 to float, and prevents the pushers 174 from exiting a respective staple retention slot 171a during transport and prior to use. An actuation sled 176 is positioned to pass longitudinally through the cartridge body 170 into engagement with the pushers 174 to sequentially eject the staples "S" from the cartridge body 170. For a detailed description of an exemplary cartridge assembly, please refer to commonly owned U.S. Pat. App. Pub. No. 2013/0098965 ("the '965 publication"), the content of which is incorporated by reference herein in its entirety.

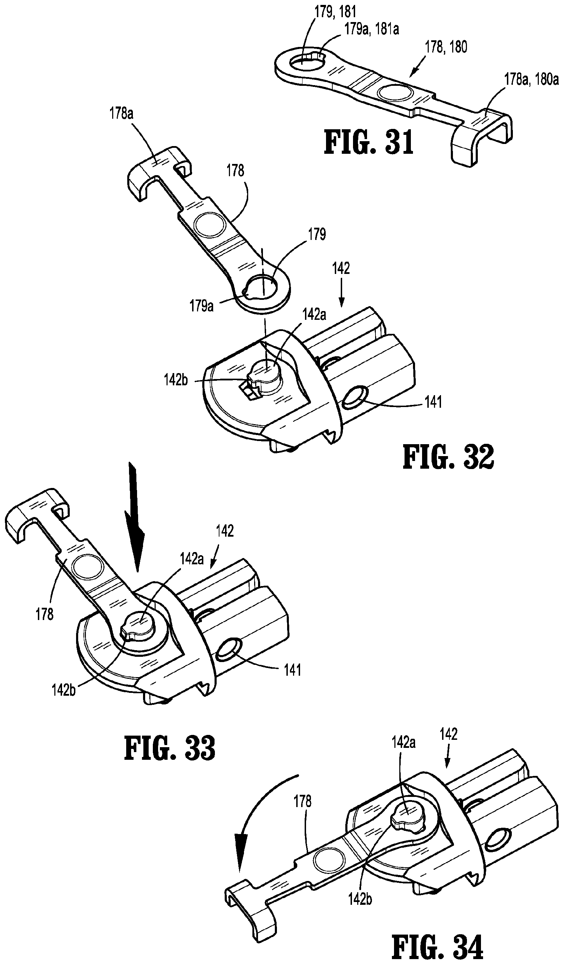

The mounting assembly 104 includes the upper mounting bracket 162 and the lower mounting member 142. Each of the upper and lower mounting brackets 162, 142 includes a pivot member or boss 162a, 142a (FIG. 33), respectively. As discussed above, the pivot member 162a is received within the hole 157 of the bracket 150a of the anvil body 150 to secure the upper mounting bracket 162 to the anvil body 150. A first coupling member 178 has a first portion which defines an opening 179 that also receives the pivot member 162a and a second portion which is received within the cutout 145a defined in the distal portion of the upper half-section 144a of the small diameter portion 112 of the body portion 102 of the stapler reload 100. The pivot member 142a on the lower mounting bracket 142 is received in an opening 181 defined in a first portion of a second coupling member 180. The second coupling member 180 has a second portion that is received within the cutout 145b defined within lower housing half-section 144b of the body portion 102 to pivotally secure the lower mounting bracket 142 to the lower housing half-section 144b of the inner body 140 of the body portion 102 of the stapler reload 100.

The pivot pins 168 extend through openings 151a formed in the anvil body 150 and openings 151b formed in the channel 164, and are received in openings 141 formed in lower mounting bracket 142 to secure the lower mounting bracket 142 to the channel 164. The upper and lower mounting brackets 162, 142 are secured together by pin members 182a, 182b.

Referring to FIGS. 5-13, a lock assembly 200 is supported on the proximal portion of large diameter portion 110 of the body portion 102 of the stapler reload 100 to prevent axial movement of the drive assembly 136 until the stapler reload 100 is properly attached to the adapter assembly 30 (FIG. 1) of the surgical stapler 10 (FIG. 1). The lock assembly 200 includes the drive block 132 supported on the proximal portion of the drive member 124 and the rotatable sleeve 134 is configured for selective engagement with the drive block 132 to prevent longitudinal movement of the drive member 124. The rotatable sleeve 134 is mounted about the proximal portion of the housing 120 of the body portion 102, and includes two proximally extending fingers 202a. The rotatable sleeve 134 also includes a distally extending flange 202b. A lock plate 204 is slideably supported within a cutout 201a of the lower housing half-section 120b of the inner housing 120 of the body portion 102 of the stapler reload 100, and is positioned to be engaged by the distally extending flange 202b of the rotatable sleeve 134 during attachment of the stapler reload 100 to the adapter assembly 30 (FIG. 1) to prevent forward movement of the drive member 124.

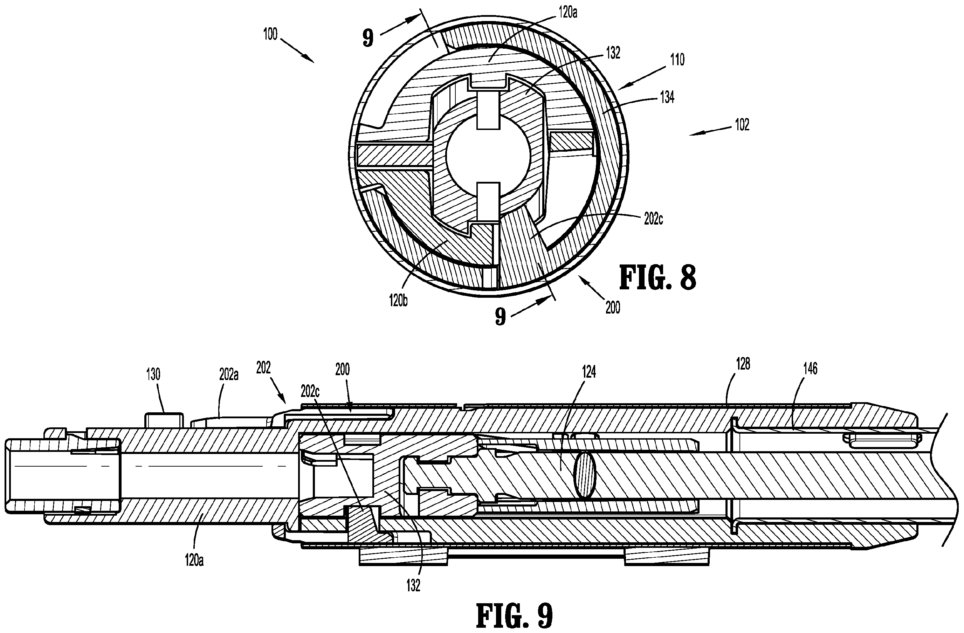

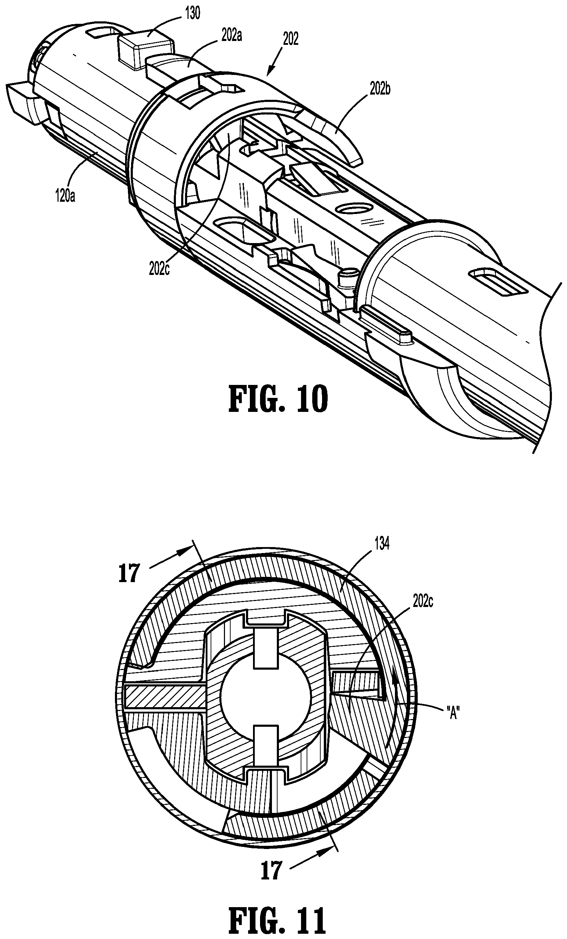

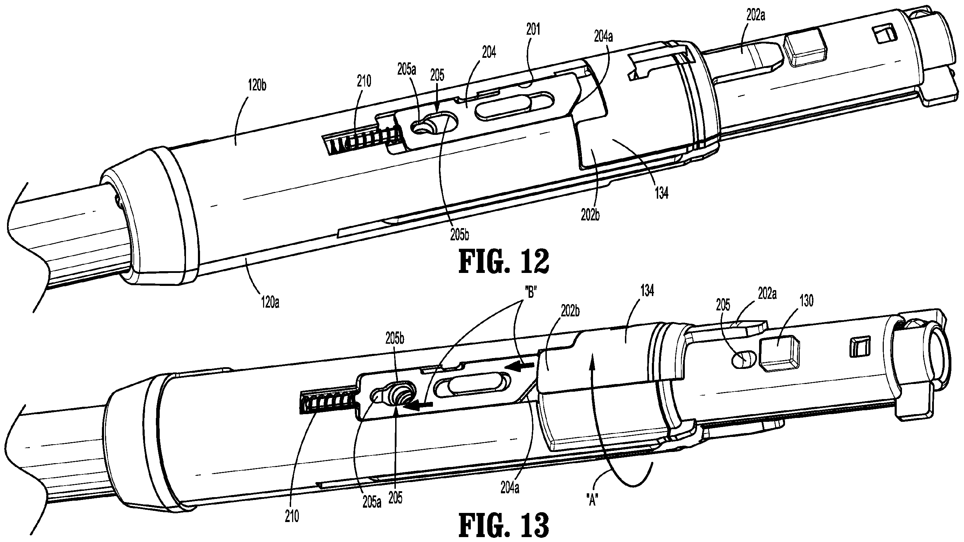

The rotatable sleeve 134 also includes a blocking finger 202c that extends downwardly into the path of the drive block 132 to obstruct movement of the drive block 132 when the rotatable sleeve 134 is in a first or locked position. When the rotatable sleeve 134 is rotated, as indicated by arrow "A" in FIGS. 11 and 13, the blocking finger 202c is moved from the first position (FIG. 8) in engagement with the drive block 132 to a second position (FIG. 11) spaced from the drive block 132 to facilitate longitudinal movement of the drive block 132 and, thus, drive member 124.

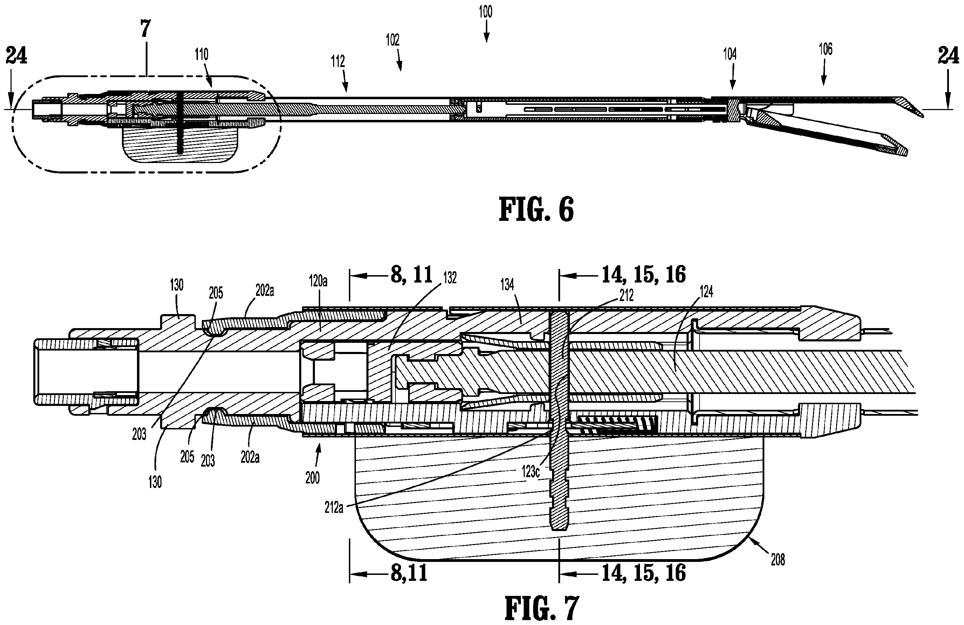

With particular reference to FIG. 7, when the rotatable sleeve 134 is positioned about the proximal portion of the lower housing half-section 120b, the proximally extending fingers 202a of the locking sleeve 134 are positioned in alignment with the nubs 130 on the upper housing half-section 120a. Each proximally extending finger 202a includes an inwardly extending protrusion 203 that is received in a respective recess 205 formed on an outer surface of the upper half section 120a to releasably retain the sleeve 134 in the first position. When the stapler reload 100 is attached to an adapter assembly 30 (FIG. 1), the proximal portion of the stapler reload 100 is inserted into the distal portion of the adapter assembly 30 (FIG. 1) and rotated to engage the bayonet-type coupling components of the stapler reload 100 and the adapter assembly 30. As the adapter assembly 30 is rotated in relation to the stapler reload 100, a portion of the adapter assembly 30 engages the proximally extending fingers 202a of the rotatable sleeve 134 to rotate the rotatable sleeve 134 about the inner housing 120 of the stapler reload 100 from the first position (FIG. 8) to the second position (FIG. 11).

The stapler reload 100 is provided with a shipping lock 208 configured to prevent operation of the stapler reload 100. The shipping lock 208 is selectively secured to the large diameter portion 110 of the body portion 102 of the stapler reload 100 and extends through an opening 123c in the drive member 124 to prevent axial movement of the drive member 124. The shipping lock 208 is configured to be separated from the body portion 102 of the stapler reload 100 only after the stapler reload 100 is properly secured to an actuation device 20 (FIG. 1).

As noted above, rotation of the rotatable sleeve 134 from the first position to the second position also causes the distally extending flange 202b of the rotatable sleeve 134 to engage the lock plate 204. With particular reference to FIGS. 12 and 13, the lock plate 204 is received within the cutout 201 of the lower housing half-section 120b and is configured to prevent release of the shipping lock 208 (FIG. 7) from engagement with the body portion 102 of the staple reload 100 until the staple reload 100 is properly secured to the adapter assembly 30 (FIG. 1). More particularly, the lock plate 204 is biased proximally by a spring 210 and defines a lock slot 205 for releasably engaging a locking pin 212 of the shipping lock 208. The lock slot 205 includes a small diameter portion 205a sized to be received around a notched portion 212a (FIG. 7) of the locking pin 212 of the shipping lock 208, and a large diameter portion 205b through which the entirety of the locking pin 212 can be received.

During rotation of the rotatable sleeve 132, the distally extending flange 206 of the rotatable sleeve 134 engages a cammed surface 204a of the lock plate 204, causing the lock plate 204 to move in the distal direction, as indicated by arrows "B" in FIG. 13, against the bias of the spring 210. As the lock plate 204 moves from a proximal position (FIG. 12) to a distal position (FIG. 13), the lock slot 205 is moved from having the small diameter portion 205a positioned about the notched portion 212a of the locking pin 212 to having the large diameter portion 205b received about the locking pin 212. With the notched portion 212a of the locking pin 212 positioned within the large diameter portion 205b, the locking pin 212 can be withdrawn from the stapler reload 100. In this manner, distal movement of the lock plate 204 permits release of the locking pin 212 of the shipping lock 208 from engagement with the stapler reload 100.

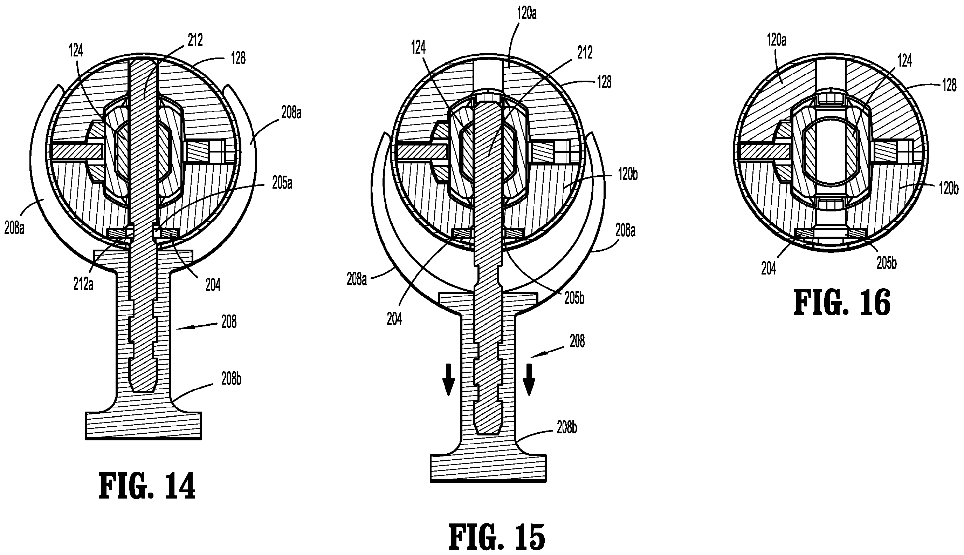

Turning now to FIG. 14, the shipping lock 208 is shown secured to the large diameter portion 110 of the body portion 102 of the stapler reload 100. In particular, curved arms 208a of the shipping lock 208 are received about proximal body tube 128 of the large diameter portion 110 of the body portion 102 and the locking pin 212 is received through openings in the upper and lower housing half-sections 120a, 120b of the housing 120, and through openings in a lockout shield 222 and the drive member 124. In this manner, the drive member 124 is prevented from longitudinal movement in any direction. The notched portion 212a of the locking pin 212 is engaged by the lock plate 204 to prevent the shipping lock 208 from accidentally disengaging from the stapler reload 100 prior to the staple reload 100 being properly secured to the adapter assembly 30 (FIG. 1).

With reference now to FIG. 15, during rotation of the rotatable sleeve 134 during attachment of the stapler reload 100 to the adapter assembly 30 (FIG. 1), the lock plate 204 is moved distally via engagement with the flange 202b of rotatable sleeve 134 (FIG. 13) to align the large portion 205b of the lock slot 205 with the locking pin 212 of the shipping lock 208. In this manner, the locking plate 204 is disengaged from within the locking pin 212 such that the locking pin 212 is no longer secured to the body portion 102 of the stapler reload 100, and the shipping lock 208 is free to be separated from the body portion 102 of the stapler reload 100. A handle portion 208b of the shipping lock 208 is configured to be grasped and facilitate separation of the shipping lock 208 from the body portion 102. More particularly, the handle portion 208b permits a user to pull the shipping lock 208 radially outward from the body portion 102 of the stapler reload 100 to overcome the spring force of the arms 208a of the shipping lock 208 to release the shipping lock 208 from the body portion 102 of the stapler reload.

Turning briefly to FIG. 16, once the shipping lock 208 (FIG. 15) is separated from the stapler reload 100, the stapler reload 100 is ready for use.

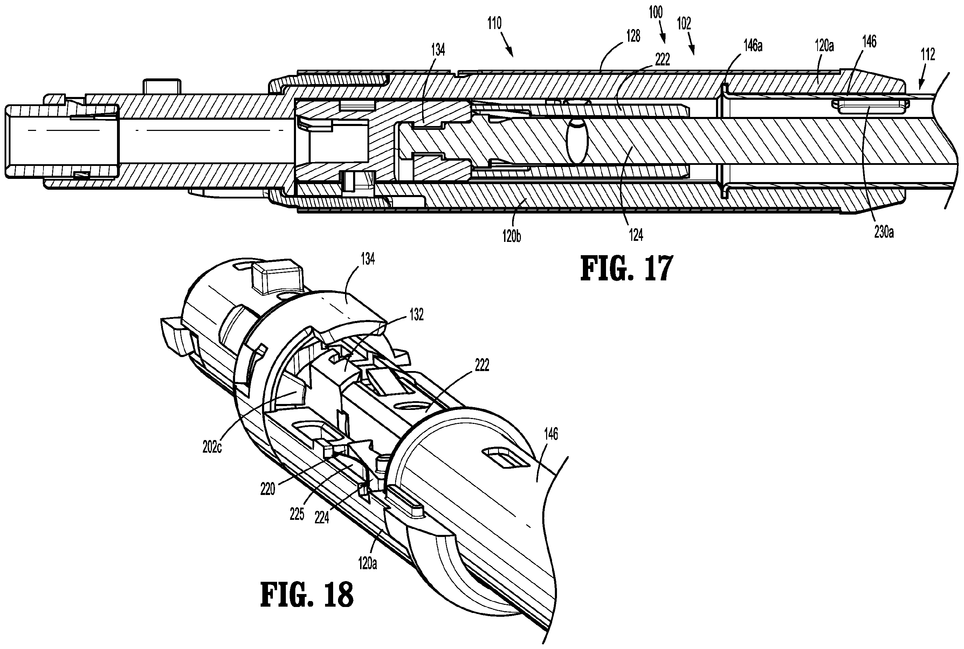

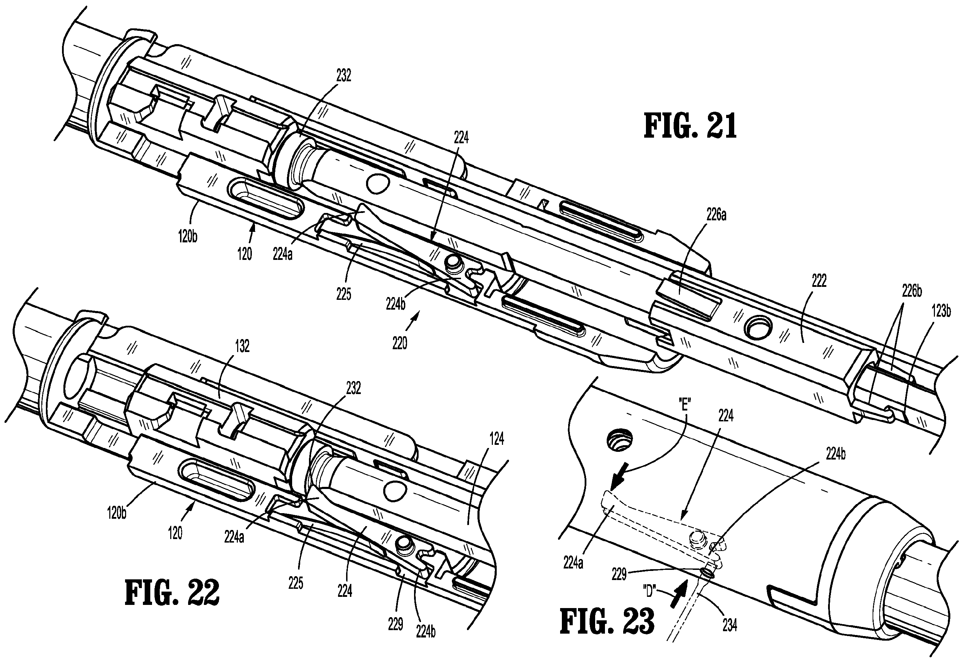

With reference now to FIGS. 5 and 18-23, the large diameter portion 110 of the body portion 102 of the stapler reload 100 includes a lockout assembly 220 to prevent subsequent advancement of the drive member 124 following firing of the stapler reload 100. The lockout assembly 220 includes a lockout shield 222 and a lockout member 224. The lockout shield 222 is slideably disposed about a proximal portion of the drive member 124 and includes a pair of proximally extending and outwardly biased shield lances 226a (FIGS. 19 and 20) and a pair of distally extending shield fingers 226b. The shield lances 226a operate to prevent retraction of the lockout shield 222 subsequent to actuation of the stapler reload 100. The shield fingers 226b engage a proximal set of notches 123a (FIG. 5) of the drive member 124 prior to actuation of the stapler reload 100, i.e., before the lockout shield 222 is moved distally (FIG. 17), to maintain the lockout shield 222 in engagement with the drive block 132. Subsequent to advancement of the drive block 132 and the lockout shield 222 (FIG. 21), the shield fingers 226b engage a distal set of notches 123b (FIG. 5) in the drive member 124 (FIG. 17) to assist in retaining the drive member 124 subsequent to retraction of the drive member 134.

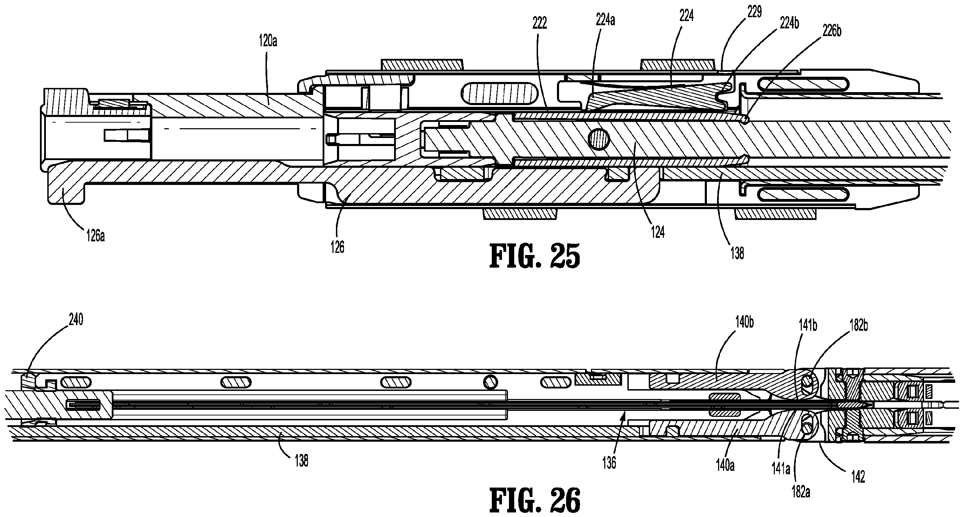

With particular reference to FIGS. 19 and 21, the lockout member 224 is pivotally supported within the inner housing 120 of the body portion 102 and includes a proximal portion 224a that is biased radially inward by a spring member, e.g., leaf spring 225, into engagement with the lockout shield 222 prior to firing of the stapler reload 100 (FIG. 26) and into engagement with the drive member 124 subsequent to retraction of the drive member 124 following actuation of the stapler reload 100.

With particular reference to FIG. 20, during actuation of the stapler reload 100, engagement of the drive block 132 by the connector rod 32 (FIG. 2) of the adapter assembly 30 (FIG. 2) causes the drive member 124 and the lockout shield 222 to advance distally, as indicated by arrows "C" in FIG. 20. As the drive block 132 is advanced, the lockout shield 222 is moved distally into the outer tube 146 of the small diameter portion 112 of the body portion 102 of the stapler reload 100. Once the lockout shield 222 is received within the outer tube 146, the shield lances 226a of the lockout shield 222 engage protrusions 230a, 230b formed on respective upper and lower housing half-sections 120a, 120b of housing 120. Engagement of the shield lances 226a with the protrusion 230a, 230b of the respective upper and lower housing half-sections 120a, 120b prevents proximal movement of the lockout shield 222 as the drive member 124 is retracted to its initial position subsequent to the actuation stroke.

With particular reference now to FIG. 21, when the drive member 124 returns to its initial position, subsequent to the actuation stroke of the stapler reload 100, as noted above, the lockout shield 222 remains within the outer tube 146 of the small diameter portion 110 of the body portion 102 of the stapler reload 100 because of the shield lances 226a of the lockout shield 222 prevent proximal movement of the lockout shield 222. Without the lockout shield 222 being received around the proximal portion of the drive member 124, the proximal portion 224a of the lockout member 224 directly engages the drive member 124. During any subsequent attempted actuations of the stapler reload 100 after the initial actuation, the proximal portion 224a of the lockout member 224 will engage a flange 232 located distal of the proximal portion of the drive member 124, thereby preventing further distal movement of the drive member 124.

As shown in FIG. 23, a lockout reset opening 229 is provided in the housing 120 of the body portion 102 of the stapler reload 100 to permit overriding of the lockout assembly 220. In particular, a tool, e.g., screw driver 234, may be received through the lockout reset opening 229 and engaged with a distal portion 224b of the lockout member 224, as indicated by arrow "D", to pivot the proximal portion 224a of the lockout member 224 from engagement with the flange 232 (FIG. 22) of the drive member 124, as indicated by arrow "E".

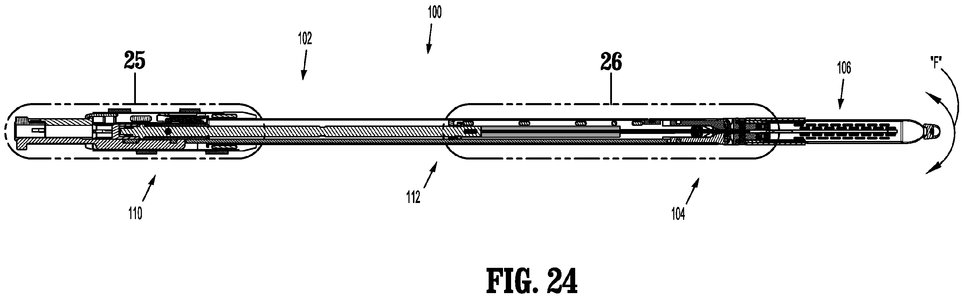

With reference briefly to FIGS. 24-26, as described above, the first articulation link 126 is releasably coupled to an articulation mechanism (not shown) of the adapter assembly 30 (FIG. 1) to control articulation of the tool assembly 106. More specifically, when the articulation mechanism of the adapter assembly 30 is operated, the first articulation link 126 is advanced (or retracted) to cause corresponding advancement (or retraction) of the second articulation link 138. The distal portion of the second articulation link 138 engages a first articulation member 140a which is pivotally connected to the pin member 182a of the lower mounting bracket 142 of the mounting assembly 104. As shown in FIG. 26, the pin member 182a is positioned at a location offset from the longitudinal axis of the body portion 102 such that longitudinal movement of the first articulation member 140a effects pivotal movement of the tool assembly 106 about a perpendicular axis, as indicated by arrow "F" in FIG. 24.

A second articulation member 140b is positioned parallel to the first articulation member 140a and engages the pin member 182b of the lower mounting bracket 142 of the mounting assembly 104. Each of the first and second articulation members 140a, 140b includes a curved support surface 141a, 141b, respectively, for supporting blow out plates 250 which extend between the body portion 102 and the tool assembly 106 of the stapler reload 100.

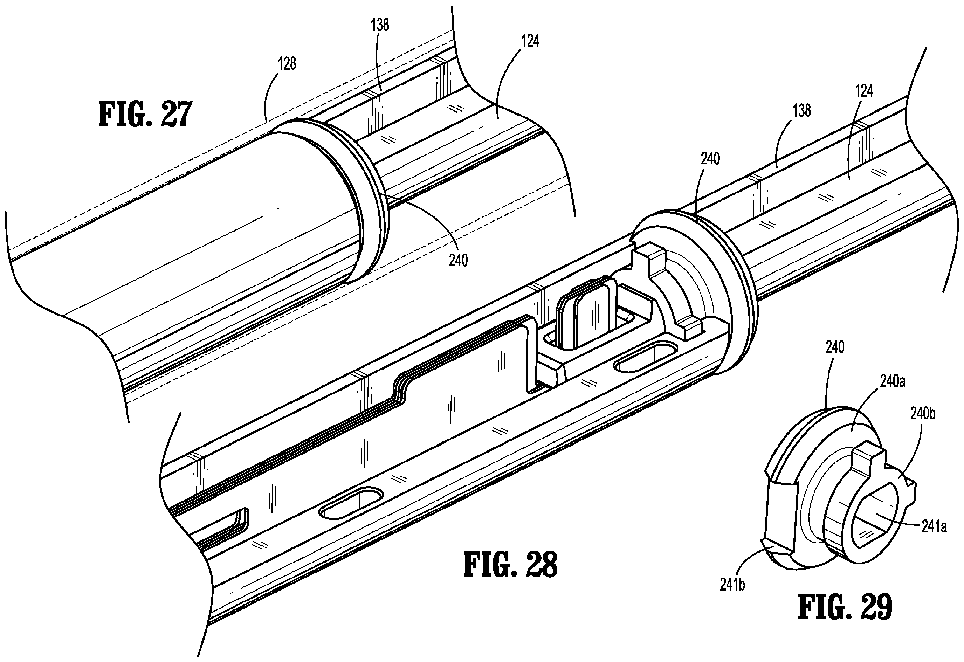

With reference to FIGS. 27-29, the stapler reload 100 includes a pnuematic seal 240 for creating a seal around the drive member 124 and the second articulation link 138. The pnuematic seal 240 includes a circular base portion 240a and a flange portion 240b extending from the base portion 240a. The base portion 240a is configured to fluidly seal a proximal portion of the inner body 144 of the small diameter portion 112 of the body portion 102 of the stapler reload 100. The flange portion 240b engages a proximal portion of the inner body 144 of the small diameter portion 112 of the body portion 102. The pnuematic seal 240 defines a central opening 241a for accommodating the drive member 124 in a sealing manner. The pnuematic seal 240 further defines a notch 241b for accommodating the second articulation link 138. The pnuematic seal 240 is disposed within the inner body 144 of the small diameter portion 112 of the body portion 102 and creates a seal between each of the drive member 124 and the second articulation link 138 and the inner body 144.

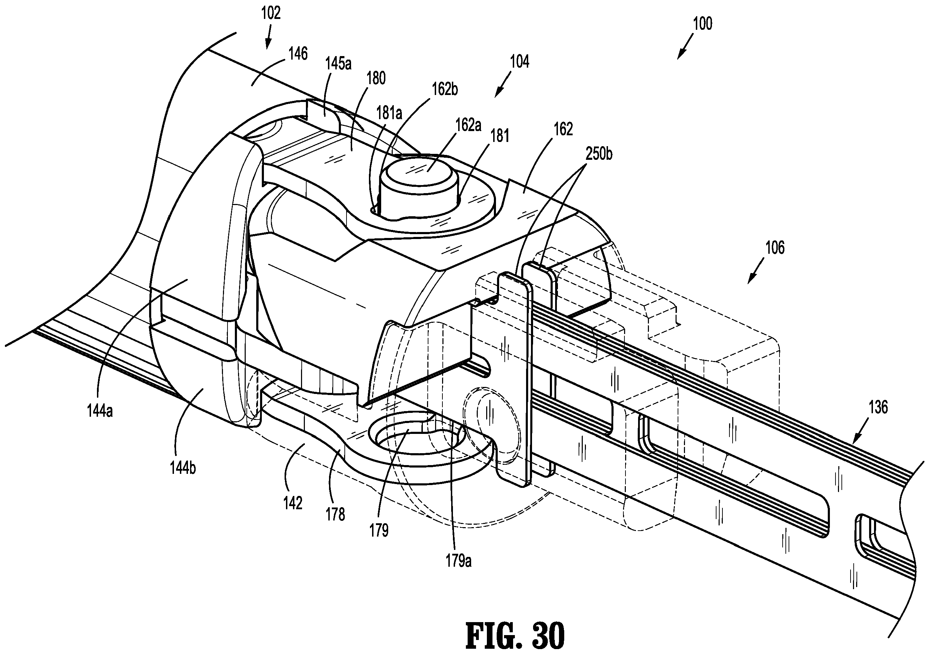

With reference now to FIGS. 30-34, the mounting assembly 104 of the stapler reload 100 includes the upper and lower mounting brackets 162, 142, and first and second coupling members 178, 180. As noted above, the first and second coupling members 178, 180 each include an opening 179, 181, respectively, for receiving pivot members 142a, 162a of respective lower and upper mounting brackets 142, 162. More particularly, each of the openings 179, 181 of the first and second coupling members 178, 180, respectively, include a notch 179a, 181a (FIG. 31) that receives the tab 142b, 162b (FIG. 30; shown in phantom) extending from the respective pivot members 142a, 162a of the upper and lower mounting brackets 162, 142. In this manner, each of the first and second coupling members 178, 180 is received about the respective pivot members 142a, 162a with the respective notches 179a, 181a of the respective first and second coupling members 178, 180 aligned with the respective tabs 142b, 162b of the respective pivot members 142a, 162a. The first and second coupling members 178, 180 are rotated from an unlocked position (FIG. 33), in which the first and second coupling members 178, 180 are disposed perpendicular to the longitudinal axis "x" of the stapler reload 100, to a locked position (FIG. 34) in which the first and second coupling members 178, 180 are disposed in alignment with the longitudinal axis "x" to secure the first and second coupling members 178, 180 to the respective lower and upper mounting brackets 142, 162. In this manner, the mounting assembly 104 creates a rivetless pivot assembly for articulating the tool assembly 106 of the stapler reload 100 relative to the body portion 102.

As described above, each of the upper and lower housing sections 120a, 120b of the body portion 102 include cutouts 145a, 145b (FIG. 5). Each of the first and second coupling members 178, 180 include folded portions 178a, 180a that are received within the cutouts 145a, 145b. The folded portions 178a, 180a of the respective first and second coupling members 178, 180 increase the strength of the engagement with the respective upper and lower half-sections 144a, 144b of the inner body 144 of the small diameter portion 112 of the body portion 102 of the stapler reload 100.

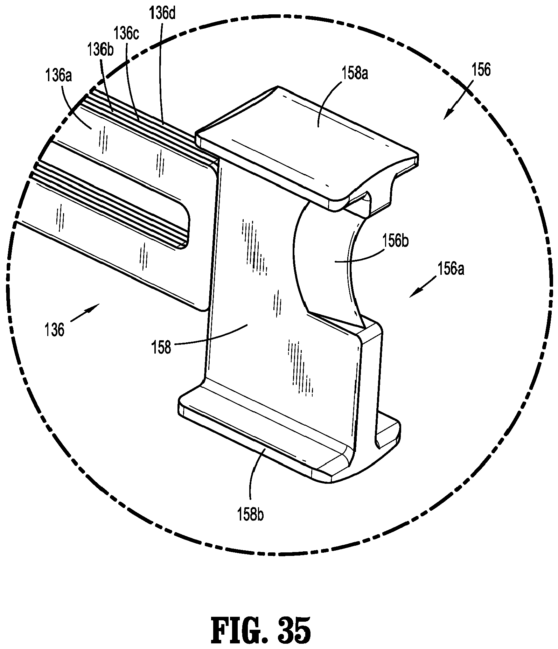

Referring to FIG. 35, in embodiments, the drive assembly 136 is formed from a plurality of stacked sheets 136a-d of a resilient material, e.g., stainless steel, spring steel. The distal portion of each of the sheets 136a-d of material of the drive assembly 136 is secured to the dynamic clamping member 156a such as by welding. The proximal ends of sheets 136a-d are hook-shaped and are received in the slot 125 in the distal portion of the drive member 124 such that longitudinal movement of the drive member 124 effects longitudinal movement of the drive assembly 136.

As best shown in FIG. 35, the dynamic clamping member 156a includes a knife 156b supported or formed on a vertical strut 158 of the dynamic clamping member 156a. The dynamic clamping member 156a includes an upper flange 158a and a lower flange 158b. The upper flange 158a is positioned to be slideably received within the cavity (not shown) of the anvil assembly 114 and the lower flange 158b is positioned to be slideably positioned along an outer surface 164a (FIG. 37) of the channel 164 of the cartridge assembly 116. In embodiments, either or both of the dynamic clamping member 156a and the channel 164 are lubricated with a hard coating. Distal movement of the drive assembly 136 initially advances the lower flange 158b into engagement with a cam surface 164b formed on the channel 164 to pivot the cartridge assembly 116 towards the anvil assembly 114 to a closed or approximated position (FIG. 38).

Thereafter, advancement of the drive assembly 136 progressively defines a maximum tissue gap between the anvil assembly 114 and cartridge assembly 116 adjacent the dynamic clamping member 156a as the dynamic clamping member 156a moves through the tool assembly 106. In embodiments, at least portions of the dynamic clamping member 156a and/or channel 164 are formed from and/or coated with a friction reducing material to minimize the force required to fire the surgical stapler 10 by facilitating substantially frictionless passage of the dynamic clamping member 156a through the stapler reload 100. For a detailed description of an exemplary drive assembly and dynamic clamping member, please refer to the '965 publication, the content of which was previously incorporated herein.

With reference to FIG. 38, a blow-out plate 250 is positioned on each side of the drive assembly 136 to prevent buckling of the drive assembly 136 during straight and articulated firing of the stapler reload 100. The blow-out plates 250 extportion between the body portion 102 and the tool assembly 106 of the stapler reload 100. A distal portion 250b of each of the blow-out plates 250 is fixedly secured to the lower mounting bracket 142 of the mounting assembly 104. In embodiments, the distal portion 250a of each of the blow-out plates 250 is press fit within a slot (not shown) formed in the lower mounting bracket 142 (shown in phantom in FIG. 30) to axially fix the distal portion 250b of each of the blow-out plates 250 to the lower mounting bracket 142. Each of the first and second articulation members 140a, 140b includes curved portions 141a, 141b, respectively, for supporting an outer surface of the blow-out plates 250.

When the drive assembly 136 is advanced to advance the dynamic clamping member 156a through the cartridge body 170 to fire staples "S" with the tool assembly 106 in an articulated position, the blow-out plates 250 prevent the drive assembly 136 from buckling outward.

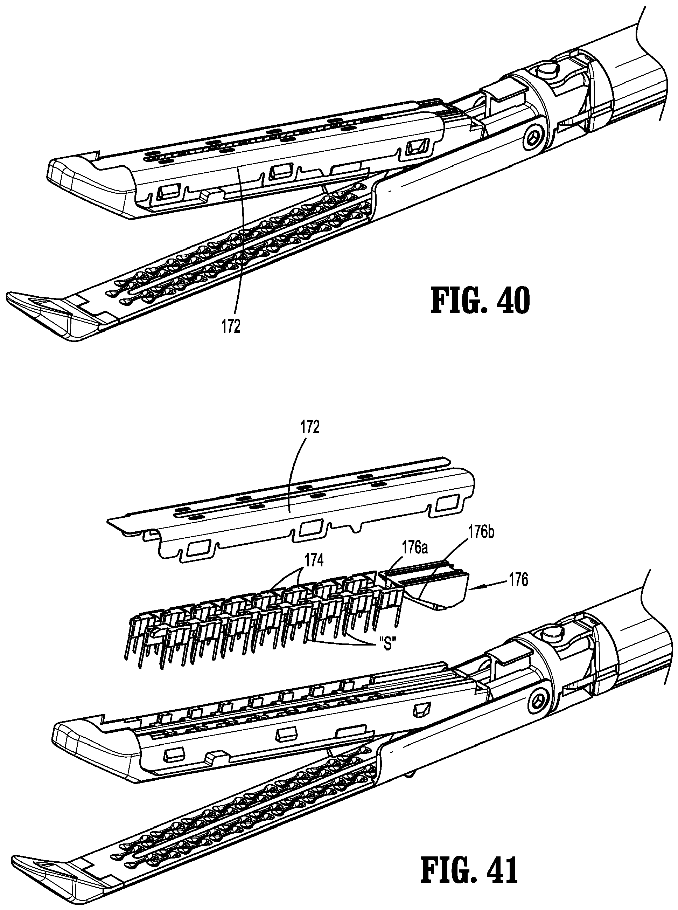

Turning to FIGS. 40 and 41, the sled 176 is supported within the cartridge body 170 at a position immediately distal of the dynamic clamping member 156a. The distal portion of the dynamic clamping member 156a is positioned to engage and drive the sled 176 through the cartridge body 170 of the cartridge assembly 116. The sled 176 includes first and second cam members 176a, 176b that are positioned to engage the pushers 174 positioned within the cartridge assembly 116 to eject the staples "S" from the cartridge body 170. Each pusher 174 supports two staples "S" positioned on one side of the knife slot 171b of the cartridge body 170. As noted above, the cartridge assembly 116 includes the cartridge shield 172 for retaining the pushers 176 within the cartridge body 170. More particularly, the cartridge shield 172 allows the pushers 176 to be floating, and prevents the pushers 174 from exiting the staple retention slots 171a during transport.

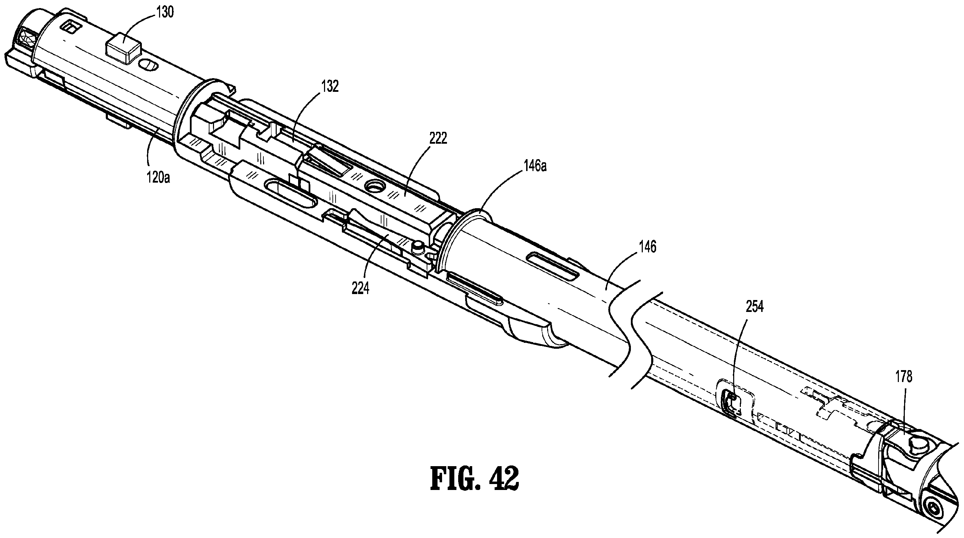

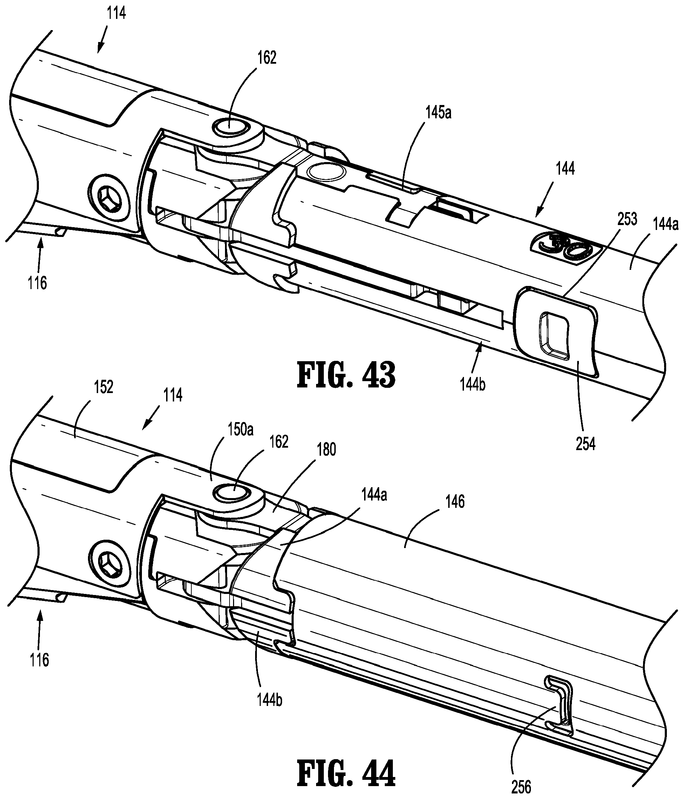

With reference now to FIGS. 42-44, the surgical stapler 10 is configured to distribute actuation forces along a load path which enables the stapler reload 100 to have a smaller diameter. The load path begins with the knife 156b (FIG. 35) of the dynamic clamping member 156a (FIG. 35) of the drive assembly 136 (FIG. 35) cutting through tissue (not shown). More specifically, in the presently disclosed surgical stapler 10, the load is transferred from the dynamic clamping member 156a to the anvil and cartridge assemblies 114, 116 as the dynamic clamping member 156a travels therethrough. The load is then transferred from the anvil and cartridge assemblies 114, 116 to the lower mounting bracket 142 by pivot pins 168. The first and second pivot members 142a, 162a of the respective lower and upper mounting brackets 142, 162 of the mounting assembly 104 transfer the load from the lower and upper mounting brackets 142, 162 to the first and second coupling members 178, 180. The first and second coupling members 178, 180 transfer the load to the inner body 144 of the small diameter portion 112 of the body portion 102 of the stapler reload 100.

With particular reference now to FIG. 42, the inner body 104 of the small diameter portion 112 of the body portion 120 of the stapler reload 100 defines a cutout 253 for receiving a dinking plate 254. The dinking plate 254 is positioned to engage a dinking lance 256 located on the outer tube 146 of the small diameter portion 112 of the body portion 102 of the stapler reload 100. The dinking plate and lance 254, 256 operate to transfer the load from the inner body 104 of the small diameter portion 112 of the body portion 102 to the inner housing 120 of the large diameter portion 110 of the stapler reload 100. The inner housing 120 transfers the load to the actuation device 20 (FIG. 1) via the adapter assembly 30 (FIG. 1).

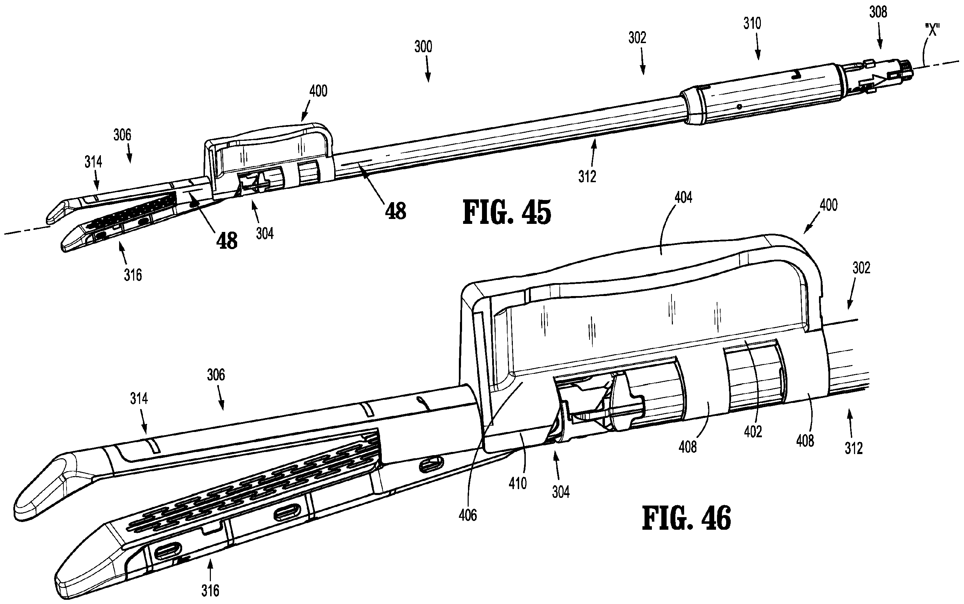

With reference now to FIGS. 45-50, another embodiment of a small diameter reload is shown generally as stapler reload 300. The stapler reload 300 is substantially similar to the stapler reload 100 described hereinabove except that the configuration of the shipping lock is changed, the location of the shipping lock is moved from the large diameter portion of the body portion to the small diameter portion of the body portion, and the configuration of the body portion and the tool assembly is modified to accommodate the changes in configuration and location of the shipping lock.

Referring initially to FIGS. 45 and 46, the stapler reload 300 includes a body portion 302, a mounting assembly 304, and a tool assembly 306. The body portion 302 defines a longitudinal axis "x" that is aligned with the longitudinal axis of the adapter assembly 30 (FIG. 1) and has a coupling portion 308, a large diameter portion 310, and an endoscopic or small diameter portion 312. The large diameter portion 310 of the stapler reload 300 has a diameter greater than the diameter of the small diameter portion 312. In embodiments, the small diameter portion 312 of the stapler reload 300 is dimensioned to be received in an 8mm trocar assembly (not shown), whereas the large diameter portion 310 is about 12mm in diameter. Alternately, other dimensions are envisioned.

The tool assembly 306 includes an anvil assembly 314 and a cartridge assembly 316. In embodiments, the cartridge assembly 316 is pivotally supported in relation to the anvil assembly 314 and is movable between an open position and a closed or approximated position. The mounting assembly 304 is supported on a distal portion of the small diameter portion 312 of the body portion 302 and pivotally supports the tool assembly 306 to facilitate articulation of the tool assembly 306 about an axis perpendicular to the longitudinal axis "x" of the body portion 302 between an articulated position in which a longitudinal axis of the tool assembly 306 defines an acute angle with the longitudinal axis "x" of the body portion 302 and a non-articulated position in which the longitudinal axes of the tool assembly 306 and the body portion 302 are aligned.

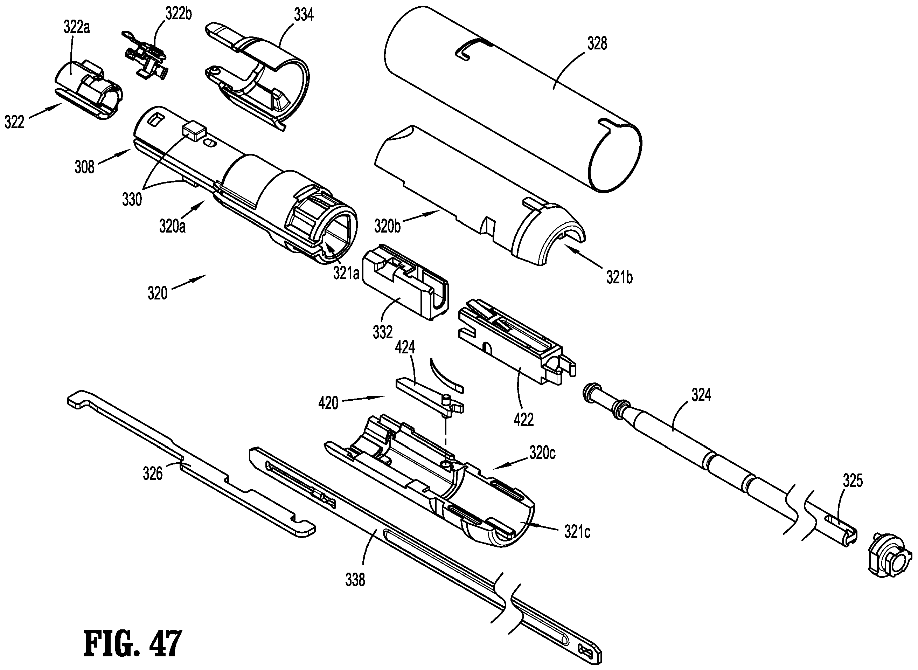

With particular reference now to FIG. 47, the large diameter portion 310 of the body portion 302 of the stapler reload 300 includes an inner housing 320 having a proximal housing section 320a, an upper housing half-section 320b, and a lower housing half-section 320c. The upper and lower housing half-sections 320b, 320c are secured relative to each other with a tab and slot configuration about a distal end of the proximal housing section 320a. Alternately, the housing sections can be secured together in other suitable manners. The proximal housing section 320a and the upper and lower housing half-sections 320b, 320c define channels 321a, 321b, 321c, respectively, which slidably receive a drive member 324 and a first articulation link 326. The proximal housing section 320a and the upper and lower housing half-sections 320b, 320c are received within a proximal body tube 328 that prevents separation of the upper and lower housing half-sections 320b, 320c and, thus, prevents separation of the upper and lower housing half-sections 320b, 320c from the proximal housing section 320a.

The proximal portion of the upper housing half-section 320a defines the coupling portion 308 and includes engagement nubs 330 for releasably engaging the distal portion of the adapter assembly 30 (FIG. 1) in a bayonet-type coupling arrangement. For a detailed description of an exemplary coupling portion please refer to the '361 patent.

An electrical connector assembly 322 is supported within the proximal portion of the upper housing half-section 320a of the body portion 302 of the stapler reload 300. The connector assembly 322 includes a connector base 322a and a contact member 322b supported on the base 322a. For a detailed discussion of an exemplary electrical connection assembly, please refer to the '929 application.

The drive member 324 of the stapler reload 300 includes a proximal portion that supports a drive block 332. The drive block 332 releasably engages a control rod 32 (FIG. 2) of the adapter assembly 30 (FIG. 2) to translate movement of the control rod 32 into movement of the drive member 324. The drive block 332 is operably engaged by a rotatable sleeve 334 to prevent firing of the stapler reload 300 prior to the stapler reload 300 being properly secured to adapter assembly 20 (FIG. 1). The drive block 332 and the rotatable sleeve 334 of the stapler reload 300 are substantially similar in structure and operation to the drive block 132 and rotatable sleeve 134 of the stapler reload 100 described hereinabove. The first articulation link 326 and a second articulation link 338 are also substantially similar in structure and operation to the first and second articulation links 126, 138 of the stapler reload 100 described hereinabove.

The large diameter portion 310 of the body portion 302 of the stapler reload 300 further includes a lockout assembly 420 to prevent readvancement of the drive member 324 following firing of the stapler reload 300. The lockout assembly 420 includes a lockout shield 422 and a lockout member 424. The lockout assembly 420 operates in a similar manner to lockout assembly 220 of the stapler reload 100 described hereinabove.

By providing the stapler reload 300 with the large diameter portion 310 for housing the drive block 332 and the rotatable sleeve 334, as well as configuring the large diameter portion 310 to accommodate components of the stapler 10, the diameter of the small diameter portion 312 is minimized, for example, to about 8mm such that it may be received within an 8mm trocar assembly (not shown), while the large diameter portion 310 may be about 12 mm in diameter or larger.

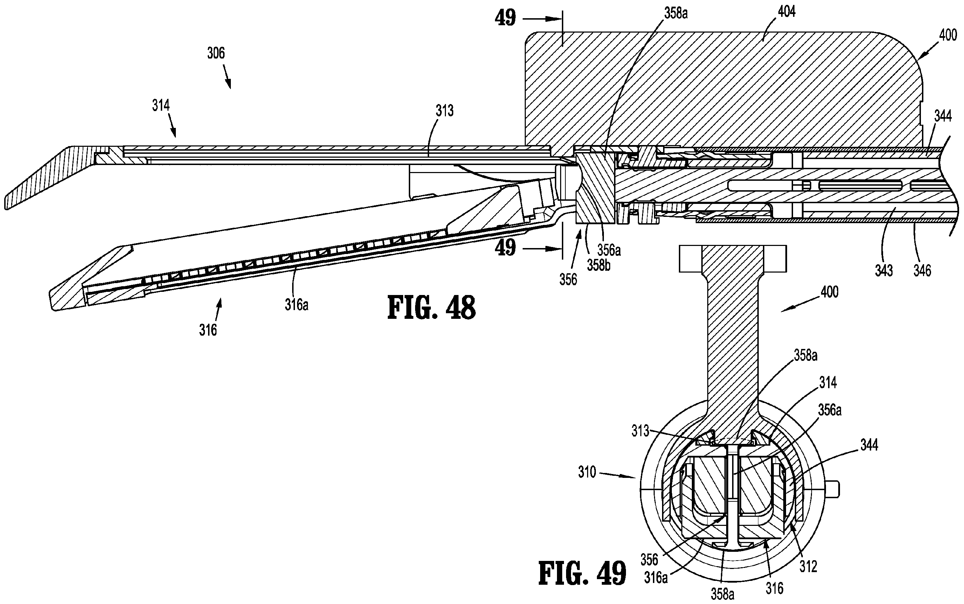

Referring now to FIGS. 48 and 49, the small diameter portion 312 of the body portion 302 of the stapler reload 300 includes an inner body 344 received within an outer tube 346. The inner body 344 defines a channel 343 that slideably receives the drive member 324 (FIG. 47) and a drive assembly 336. A distal end of the drive assembly 336 includes a dynamic clamping member 356. The dynamic clamping member 356 includes a knife portion 356a and upper and lower flanges 358a, 358b. The upper flange 358a is positioned to be slideably received within a longitudinal cavity 313 of the anvil assembly 314 and the lower flange 358b is positioned to be slideably positioned along an outer surface 316a of the cartridge assembly 316. As described in detail above with regard to the stapler reload 100, initial distal movement of the drive assembly 336 causes pivoting of the cartridge assembly 316 towards the anvil assembly 314 to a closed or approximated position and continued distal movement of the drive assembly 336 within the tool assembly 306 effects the stapling and cutting of tissue (not shown).

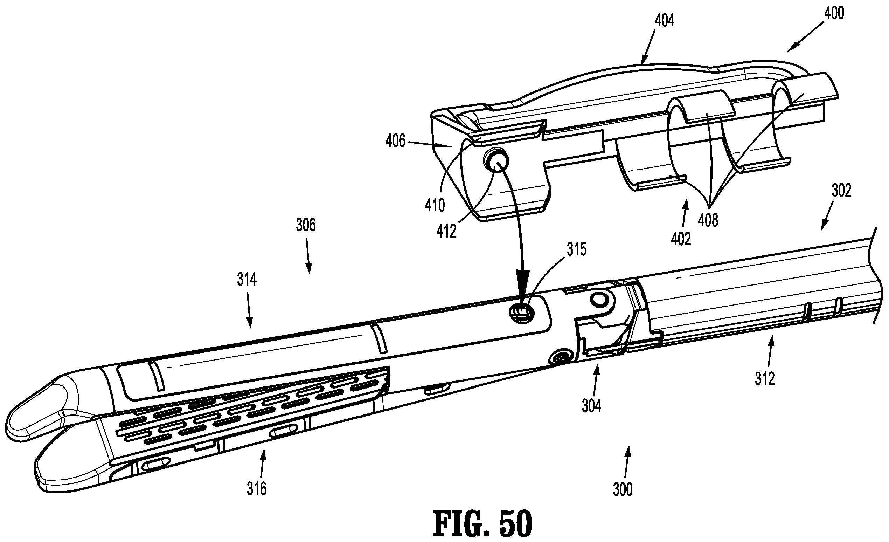

With additional reference to FIG. 50, the stapler reload 300 is provided with a shipping lock 400 configured to prevent operation of the stapler reload 300 when the shipping lock 400 is secured to the stapler reload 300. The shipping lock 400 is releasably secured to the small diameter portion 312 of the body portion 302 of the staple reload 300 and extends across the mounting assembly 304 and over a proximal portion of the tool assembly 306. The shipping lock 400 is configured to prevent advancement of the drive assembly 336 of the staple reload 300 and to prevent articulation of the tool assembly 306 relative to the body portion 302.

The shipping lock 400 includes an engagement portion 402, a handle portion 404, and a locking portion 406. The engagement portion 402 of the shipping lock 400 releasably secures the shipping lock 400 to the small diameter portion 312 of the body portion 302 of the stapler reload 300. More particularly, the engagement portion 402 includes a plurality of resilient, curved arms 408 that operate together to releasably grasp the small diameter portion 312 of the body portion 302. Although shown with two sets of opposed curved arms 408, it is envisioned that the shipping lock 400 may include any number of curved arms 408.

The handle portion 404 of the shipping lock 400 is configured to be grasped by a user and facilitate separation of the shipping lock 400 from the body portion 302 of the stapler reload 300. More particularly, the handle portion 404 of the shipping lock 400 permits the user to pull the shipping lock 400 radially outward from the body portion 302 of the stapler reload 300 to overcome the resilient force of the engagement portion 402 of the shipping lock 400 to release the shipping lock 400 from the body portion 302 of the stapler reload 300.

The locking portion 406 of the shipping lock 400 includes a locking flange 410 and a locking projection 412. The locking flange 410 is received about a proximal portion of the anvil assembly 314 of the tool assembly 306 to prevent articulation of the tool assembly 306 relative to the body portion 302. The locking projection 412 is configured to extend through an opening 315 in the proximal portion of the anvil assembly 314 into engagement with the dynamic clamping member 356 when the shipping lock 400 is secured to the stapler reload 300 to prevent advancement of the drive assembly 336. More particularly, the locking projection 412 of the shipping lock 400 engages the upper flange 358a of the dynamic clamping member 356 of the drive assembly 336 to prevent distal movement of the drive assembly 336.

Once the shipping lock 400 is separated from the stapler reload 300, the stapler reload 300 may be used in as described above with reference to the stapler reload 100.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A surgical stapler comprising:
   an actuation device;
   a stapler reload releasably secured to the actuation device, the stapler reload including a body portion, a tool assembly, and a drive assembly movable within tool assembly, the body portion including a large diameter portion defining a first diameter and a small diameter portion defining a second diameter extending distally from the large diameter portion, the tool assembly being supported on a distal portion of the small diameter portion, wherein the small diameter portion is dimensioned to pass through an 8mm trocar; and
   a shipping lock releasably secured to the distal portion of the small diameter portion of the body portion and engagable with the drive assembly for preventing longitudinal movement of the drive assembly.
2. The surgical stapler according to paragraph 1, wherein the shipping lock is configured to prevent articulation of the tool assembly relative to the body portion.
3. The surgical stapler according to paragraph 2, wherein the shipping lock includes a locking portion that engages the tool assembly to prevent articulation of the tool assembly relative to the body portion.
4. The surgical stapler according to paragraph 1, wherein the tool assembly includes an anvil assembly and a cartridge assembly supporting a plurality of staples and the drive assembly is movable through the tool assembly to eject the plurality of staples from the cartridge assembly.
5. The surgical stapler according to paragraph 1, wherein the drive assembly includes a clamping member and the shipping lock includes a projection, the projection engaging the clamping member when the shipping lock is secured to the body portion to obstruct advancement of the drive assembly.
6. The surgical stapler according to paragraph 5, wherein the clamping member includes an upper flange, the projection of the shipping lock engaging the upper flange to prevent advancement of the drive assembly.
7. The surgical stapler according to paragraph 1, wherein the shipping lock includes curved arms configured to frictionally engage the body portion of the stapler reload.
8. A surgical stapler comprising:
   an elongate body;
   a tool assembly pivotally secured to the elongate body;
   a drive member movable within the tool assembly between retracted and advanced positions; and
   a shipping lock releasably secured to the elongate body, the shipping lock including a projection, wherein when the shipping lock is secured to the elongate body, the projection obstructs movement of the drive member to its advanced position.
9. The surgical stapler according to paragraph 8, wherein the shipping lock is configured to prevent the tool assembly from pivoting relative to the body portion.
10. The surgical stapler according to paragraph 9, wherein the shipping lock includes a locking portion that engages the tool assembly to prevent pivoting of the tool assembly relative to the body portion.
11. The surgical stapler according to paragraph 8, wherein the tool assembly includes an anvil assembly and a cartridge assembly supporting a plurality of staples and the drive assembly is movable through the tool assembly to eject the plurality of staples from the cartridge assembly.
12. The surgical stapler according to paragraph 8, wherein the drive assembly includes a clamping member, wherein the projection is positioned to obstruct movement of the clamping member towards the advanced position when the shipping lock is secured to the body portion.
13. The surgical stapler according to paragraph 12, wherein the clamping member includes an upper flange, the projection of the shipping lock being positioned to engage the upper flange to obstruct movement of the drive member to the advanced position.
14. The surgical stapler according to paragraph 8, wherein the shipping lock includes curved arms configured to frictionally engage the elongate body.

## Claims

1. A stapler reload (300) comprising:
a body portion (302), a tool assembly (306), and a mounting assembly (304); drive assembly (336) movable within tool assembly, the body portion (302) including a coupling portion (308), a large diameter portion (310) defining a first diameter and a small diameter portion (312) defining a second diameter extending distally from the large diameter portion, the tool assembly (306) being supported on a distal portion of the small diameter portion (312), wherein the small diameter portion is dimensioned to be received in an 8mm trocar; and
a shipping lock (400) releasably secured to the distal portion of the small diameter portion (312) of the body portion (302) and extending across the mounting assembly (304) and over a proximal portion of the tool assembly (306), said shipping lock (400) configured to be engagable with the drive assembly (336) for preventing longitudinal movement of the drive assembly (336),said shipping lock (400) comprising an engagement portion (402), a handle portion (404),and a locking portion (406), the engagement portion (402) configured to releasably secure the shipping lock (400) to the small diameter portion (312) of the body portion (302) of the stapler reload (300), said engagement portion (402)comprising a plurality of resilient, curved arms (408) are configured to operate together to releasably grasp the small diameter portion (312) of the body portion (302).

2. The stapler reload (300) according to claim 1, wherein the tool assembly (306) includes an anvil assembly (314) and a cartridge assembly (316) supporting a plurality of staples and the drive assembly (336) is movable through the tool assembly to eject the plurality of staples from the cartridge assembly (316).

3. The stapler reload (300) according to claim 2, wherein the distal end of the drive assembly (336) comprises a dynamic clamping member (356), said dynamic clamping member (356) comprising a knife portion (356a) and upper and lower flanges (358a, 358b), the upper flange (358a) configured to be positioned to be slideably received within a longitudinal cavity (313) of the anvil assembly (314) and the lower flange (358b) configured to be is positioned to be slideably positioned along an outer surface (316a) of the cartridge assembly (316).

4. The stapler reload (300) according to any preceding claim, wherein the handle portion (404) of the shipping lock (400) is configured to be grasped by a user and facilitate separation of the shipping lock (400) from the body portion (302) of the stapler

5. The stapler reload (300) according to claim 2 or any claim dependent thereon, wherein the locking portion (406) of the shipping lock 400 comprises a locking flange (410) and a locking projection (412), said locking flange (410) configured to be received about a proximal portion of the anvil assembly (314) of the tool assembly (306) to prevent articulation of the tool assembly (306) relative to the body portion (302).

6. The stapler reload according to claim 5, wherein the locking projection (412) is configured to extend through an opening (315) in the proximal portion of the anvil assembly (314) into engagement with the dynamic clamping member (356) when the shipping lock (40) is secured to the stapler reload (300) to prevent advancement of the drive assembly (336).

7. The stapler reload (300) according to any preceding claim, comprising at drive member (324), said drive member (324) comprising a proximal portion that supports a drive block (332), said drive block (332) configured to be releasably engageable with a control rod (32) of and adapter assembly (30) to translate movement of the control rod (32) into movement of the drive member (324).

8. The stapler reload (300) according to claim 7, wherein the drive block (332) is operably engaged by a rotatable sleeve (334) to prevent firing of the stapler reload 300 prior to the stapler reload 300 being properly secured to the adapter assembly (30).

9. The stapler reload (300) according to any preceding claim, wherein the mounting assembly (304) is supported on a distal portion of the small diameter portion (312) of the body portion (302) and pivotally supports the tool assembly (306).

10. The stapler reload (300) according to claim 9, wherein the mounting assembly (304) is configured to facilitate articulation of the tool assembly (306) about an axis perpendicular to the longitudinal axis (x) of the body portion (302) between an articulated position in which a longitudinal axis of the tool assembly (306) defines an acute angle with the longitudinal axis (x) of the body portion (302) and a non-articulated position in which the longitudinal axes of the tool assembly (306) and the body portion (302) are aligned.

11. The stapler reload (300) according to any preceding claim, wherein the large diameter portion (310) of the body portion (302) includes an inner housing (320) having a proximal housing section (320a), an upper housing half-section (320b), and a lower housing half-section (320c), wherein the proximal housing section (320a) and the upper and lower housing half-sections (320b, 320c) define channels (321a, 321b, 321c) which slidably receive a drive member (324) and a first articulation link (326), preferably wherein the proximal housing section (320a) and the upper and lower housing halfsections (320b, 320c) are received within a proximal body tube (328) that prevents separation of the upper and lower housing half-sections (320b, 320c) and, prevents separation of the upper and lower housing half-sections (320b, 320c) from the proximal housing section (320a).

12. The stapler reload (300) according to claim 11, wherein the proximal portion of the upper housing half-section (320a) defines the coupling portion (308) and includes engagement nubs (330) for releasably engaging the distal portion of an
adapter assembly (30), preferably in a bayonet-type coupling arrangement.

13. The stapler reload (300) according to any preceding claim, further comprising an electrical connector assembly (322) supported within the proximal portion of the upper housing half-section (320a) of the body portion (302), said connector assembly (322) including a connector base (322a) and a contact member (322b) supported on the base (322a).

14. The stapler reload (300) according to any preceding claim, wherein the large diameter portion (310) of the body portion (302) further includes a lockout assembly (420) configured to prevent readvancement of the drive member (324) following firing of the stapler reload (300), said lockout assembly (420) comprising a lockout shield (422) and a lockout member (424).

15. A surgical stapler comprising:
A handle assembly
and
a stapler reload (300) according to any preceding claim.
